# EUROPEAN PATENT APPLICATION

(11) **EP 3 232 198 A1**
(43) Date of publication of application: **18.10.2017**
(21) Application number: 15868405.0
(22) Date of filing: 07.12.2015
(51) Int. Cl.: G01N 33/574, G01N 33/533, C12Q 1/68

(54) **BIOMARKER FOR DIAGNOSIS OF HEPATOMA AND USE THEREOF**

(30) Priority: 12.12.2014 KR 20140179004; 04.12.2015 KR 20150172020
(71) Applicant: Seoul National University R&DB Foundation, Gwanak-gu Seoul 08826 (KR)
(72) Inventor: KIM, Youngsoo, Seoul 06284 (KR); YOON, Junghwan, Seoul 03080 (KR); KIM, Hyunsoo, Bucheon si Gyeonggi-do 14459 (KR)
(74) Representative: Klunker IP Patentanwälte PartG mbB
(86) International application number: PCT/KR2015/013296
(87) International publication number: WO 2016/093567

(57) **Abstract**

Disclosed in the present application is a marker or a marker combination enabling diagnosis, prognosis, or treatment monitoring of hepatoma with high accuracy and sensitivity. The marker according to the present application enables simple and effective diagnosis or monitoring of hepatoma by a noninvasive test using blood, thus can be developed into diverse test kits, and can be very usefully utilized in early detection of hepatoma at home or in general practice, for example, through POCT development.

## Description

The present invention relates to a biomarker for diagnosis of hepatoma and use thereof.

Hepatoma or hepatocellular carcinoma, HCC, is the most common type of liver cancer in adults and the third leading cause of cancer death (Stefaniuk P, et al., 2010, World J Gastroenterol 16: 418-424). Hepatoma is a disease of which symptoms do not show until the disease has progressed significantly. For this reason, many patients miss ideal treatment opportunities and even when they do not, they have extremely poor prognoses. In particular, it is a fatal disease of which patients without resection may die within a year. In consideration of the clinical circumstances, a technology that enables early diagnosis of cancer and prognosis prediction is the most realistic alternative solution for cancer treatment and diagnosis.

Particularly, the HCC is known to frequently occur in patients who have risk factors such as hepatitis viruses, alcohol abuse, or cirrhosis and target population of patients at high risk to which selective surveillance will be conducted by using a biomarker for early diagnosis is clear. In fact, early diagnosis conducted every six months appeared to increase hepatoma patients' survival rate and reduce their mortality rate by 37%. A biomarker test that can accurately diagnose hepatoma early in normal persons has not been developed yet and a test method used for non-invasively diagnosing hepatoma early in a high-risk group is a serum alpha-fetoprotein test (AFP). At the time of development, the AFP suggested 20ng/mL as its base level that may achieve both sensitivity and specificity at a good level but in this case, the sensitivity reaches just 60% and if hepatoma is diagnosed based on 200ng/mL under the current guideline for diagnosing hepatoma of the international conference, specificity increases but sensitivity merely amounts to 22%. As a result of the existing studies, the AFP, in general, is well known to have approximate sensitivity of 66% and specificity of 82%, and therefore, it has a limitation in diagnosing all patients with hepatoma.

Although not included in diagnostic criteria, serum biomarkers helpful for diagnosis of hepatoma include descarboxyprothrombin (DCP), prothrombin induced by vitamin K absence-II (PIVKA-II), a ratio of glycosylated AFP (L3 fraction) to total AFP, alpha-fucosidase, glypican 3, HSP-70, and etc. However, most of them are useful as prognostic factors and not used for selective surveillance because they have low accuracy when used separately. It is determined that it has reached the limit in early diagnosis of hepatoma with a single serum biomarker. In addition, patients diagnosed with the disease at the stage which may be treated with radical treatments such as surgery or high frequency wave thermal therapy account for just 30% of all patients with hepatoma.

U.S. Patent Laid-Open Publication No. 2010/0304372 relates to a method and a composition for diagnosing hepatoma, and discloses the method for diagnosing hepatoma by detecting a CpG island present in BASF1, SPINT2, APC, CCND2, CFTR, RASSF1, and SRD5A2 genes.

Korean Patent Registration No. 1161789 relates to new biomarkers for hepatoma and their uses, and discloses biomarkers for diagnosing or prognosing hepatoma by detecting NK4 protein, etc.

Accordingly, it is urgent to develop biomarkers capable of early diagnosis of hepatoma at a stage where radical treatments are possible, with improved specificity and sensitivity, overcoming limitations of individual tests.

It is an object of the present invention to provide biomarkers for diagnosing hepatoma.

In accordance with one aspect of the present invention, there is provided a composition for diagnosing or prognosing hepatoma, including; a detection reagent of at least one biomarker selected from a group consisting of Insulin-like growth factor-binding protein complex acid labile subunit (ALS), Alpha-1-microglobulin/bikunin precursor (AMBP), Carboxypeptidase B2 (CPB2), Cholinesterase (CHLE), Clusterin (CLUS), Beta-Ala-His dipeptidase (CNDP1), Complement component C6 (CO6), Carboxypeptidase N subunit 2 (CPN2), Coagulation factor XI (FA11), Fibronectin (FINC), Hepatocyte growth factor activator (HGFA), Insulin-like growth factor-binding protein 3 (IBP3), Insulin-like growth factor II (IGF2), inter-alpha-trypsin inhibitor heavy chain H1 (ITIH1), Kallistatin (KAIN), Plasma kallikrein (KLKB1), Phosphatidylcholine-sterol acyltransferase (LCAT), Plasminogen-related protein A (PLGA), Plasminogen (PLMN), Prothrombin (THRB), Vitamin D binding protein (VTDB), and Vitronectin (VTNC).

In accordance with another aspect of the present invention, there is provided a method for detecting an *in vitro* biomarker of hepatoma to provide information required to diagnose or prognose the hepatoma, including steps of: detecting presence and/or concentration of a nucleic acid and/or protein of at least one biomarker selected from a group consisting of Insulin-like growth factor-binding protein complex acid labile subunit (ALS), Alpha-1-microglobulin/bikunin precursor (AMBP), Carboxypeptidase B2 (CPB2), Cholinesterase (CHLE), Clusterin (CLUS), Beta-Ala-His dipeptidase (CNDP1), Complement component C6 (CO6), Carboxypeptidase N subunit 2 (CPN2), Coagulation factor XI (FA11), Fibronectin (FINC), Hepatocyte growth factor activator (HGFA), Insulin-like growth factor-binding protein 3 (IBP3), Insulin-like growth factor II (IGF2), inter-alpha-trypsin inhibitor heavy chain H1 (ITIH1), Kallistatin (KAIN), Plasma kallikrein (KLKB1), Phosphatidylcholine-sterol acyltransferase (LCAT), Plasminogen-related protein A (PLGA), Plasminogen (PLMN), Prothrombin (THRB), Vitamin D binding protein (VTDB), and Vitronectin (VTNC) from a biological sample derived from a subject; comparing a result from detecting the level or the presence of the nucleic acid or the protein with a result corresponding to a biomarker of a sample of a control group; and determining as the hepatoma if there is any change in the concentration or any change in a state of the presence of the nucleic acid or the protein of the sample of the subject in comparison with the sample of the control group.
Fig. 1 schematically represents an MRM analysis process used for researching and analyzing biomarkers.
Fig. 2 represents whether endogenous peptides in blood and synthetic peptides are co-eluted or not.
Fig. 3 represents Q3 intensity patterns of endogenous peptides in the blood and synthetic peptides.
Fig. 4 represents a method for confirming an endogenous peptide in the blood.
Fig. 5 illustrates a result of measuring concentration of an endogenous peptide in the blood, as dynamic range distribution.
Fig. 6 represents levels in the blood for low and high abundance targets.
Fig. 7 represents a method for determining infusion concentration of SIS peptide.
Fig. 8 represents a result of implementing a panel of multi-protein biomarkers in an LC group vs. an HCC group.
Fig. 9 represents a result of implementing a panel of multi-protein biomarkers in an HCC group vs. a recovery group.

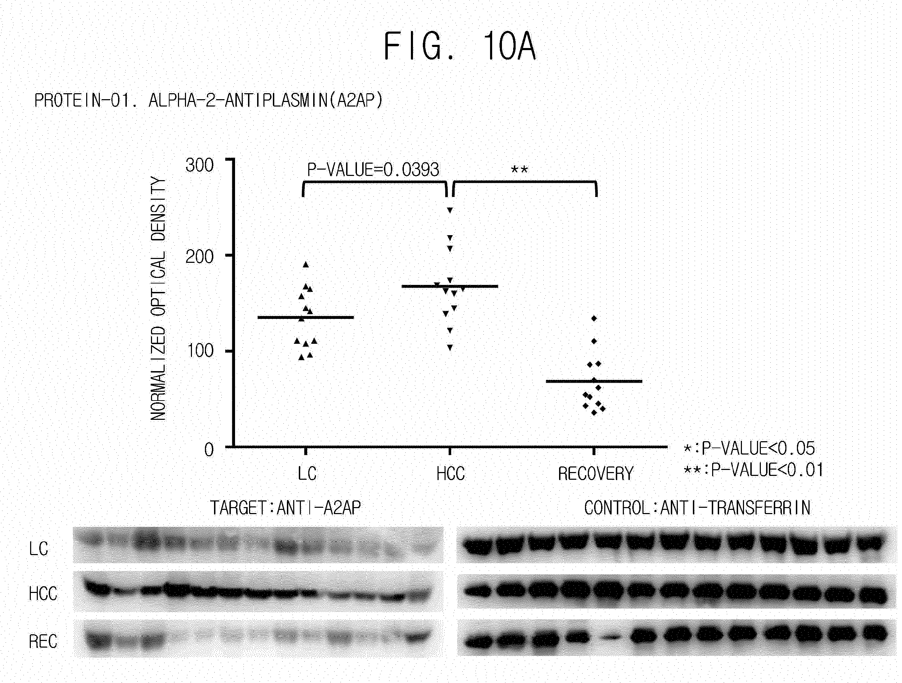

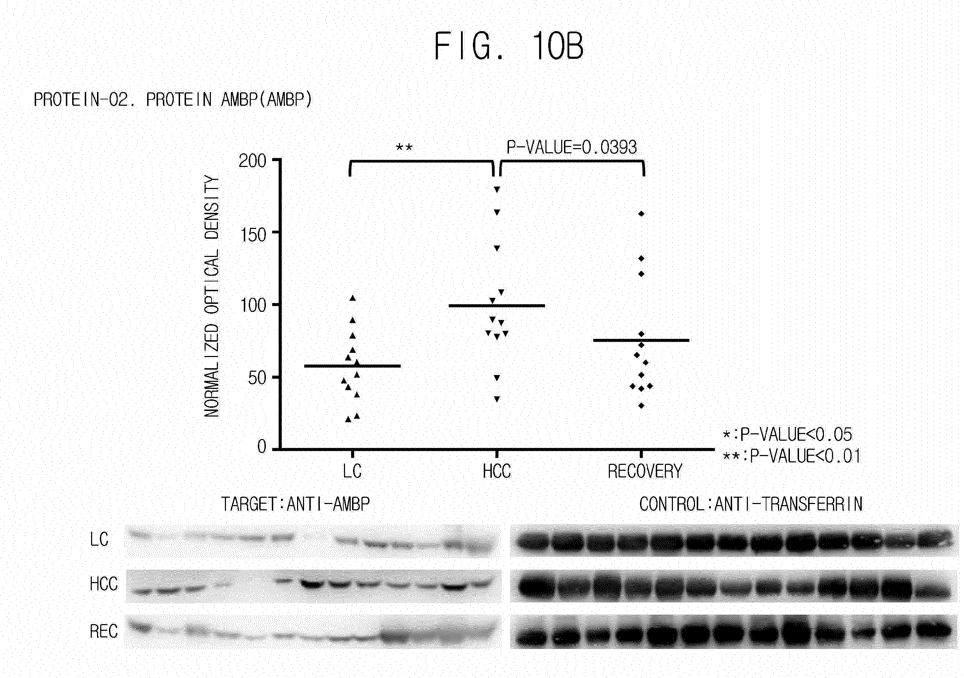

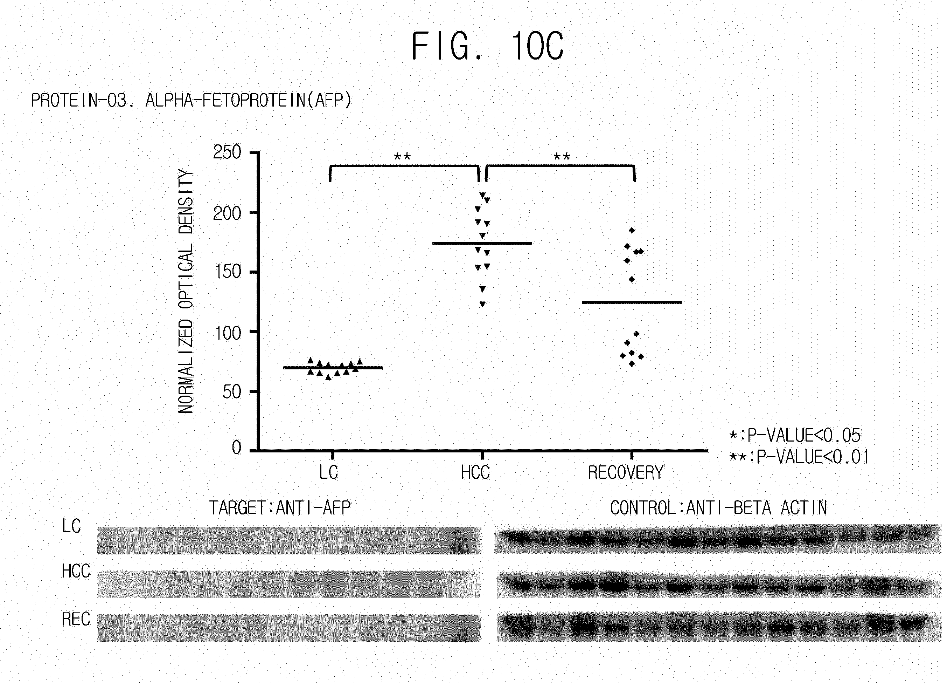

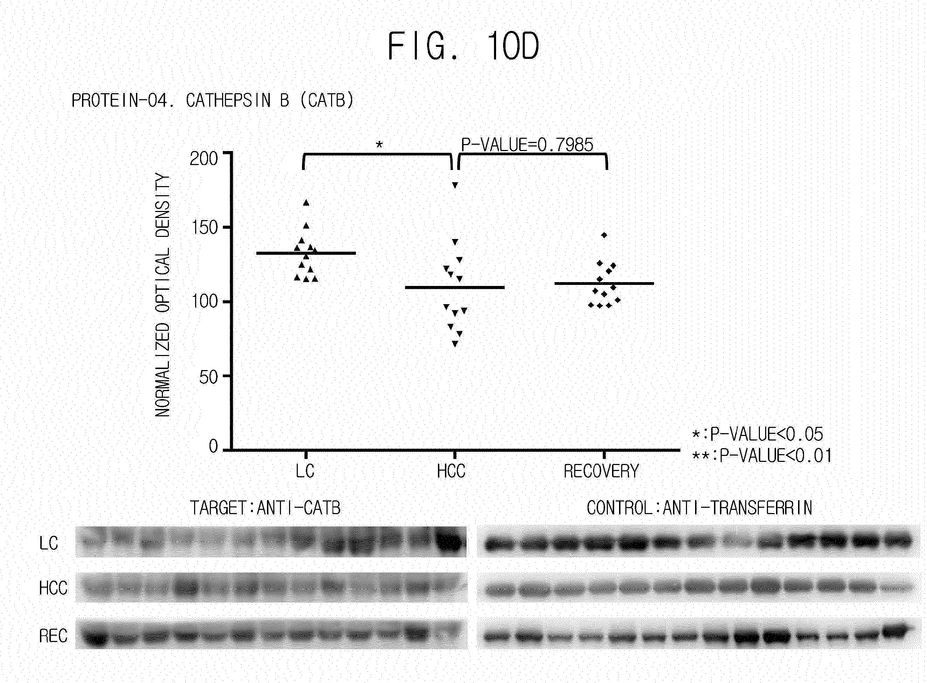

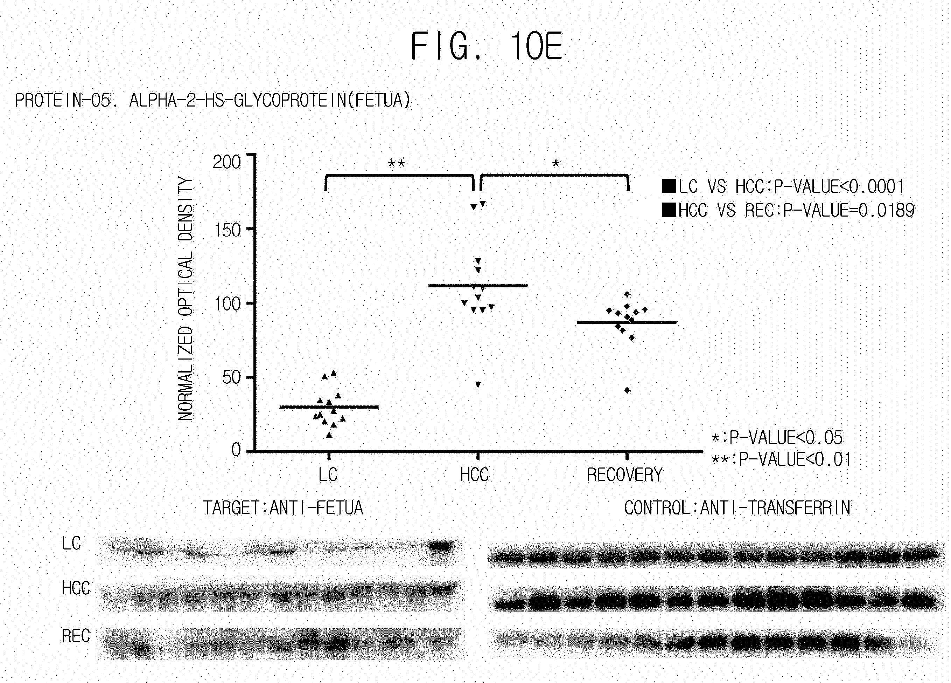

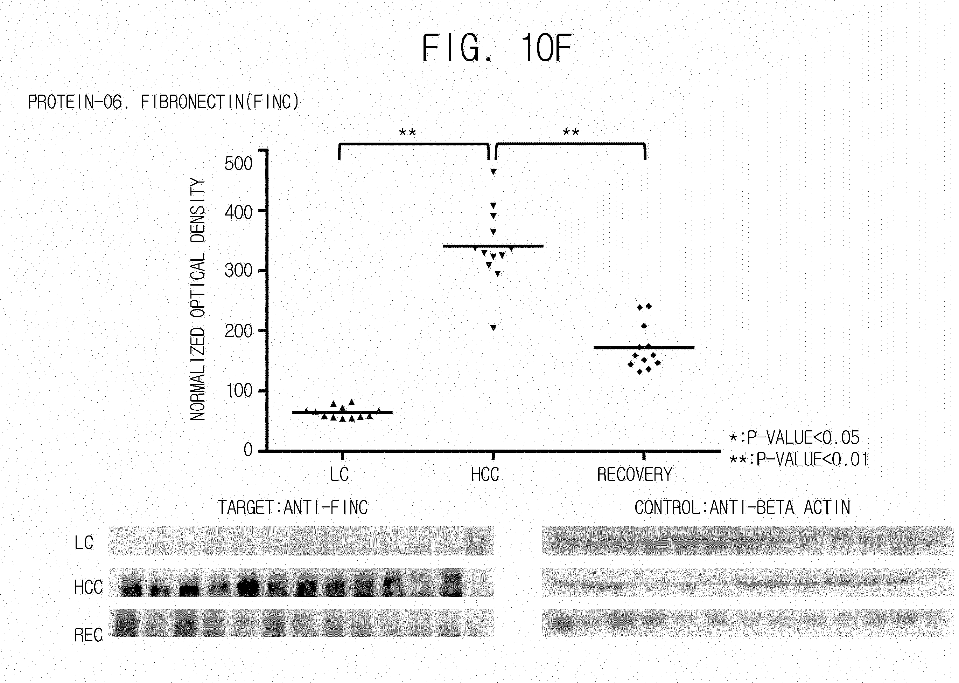

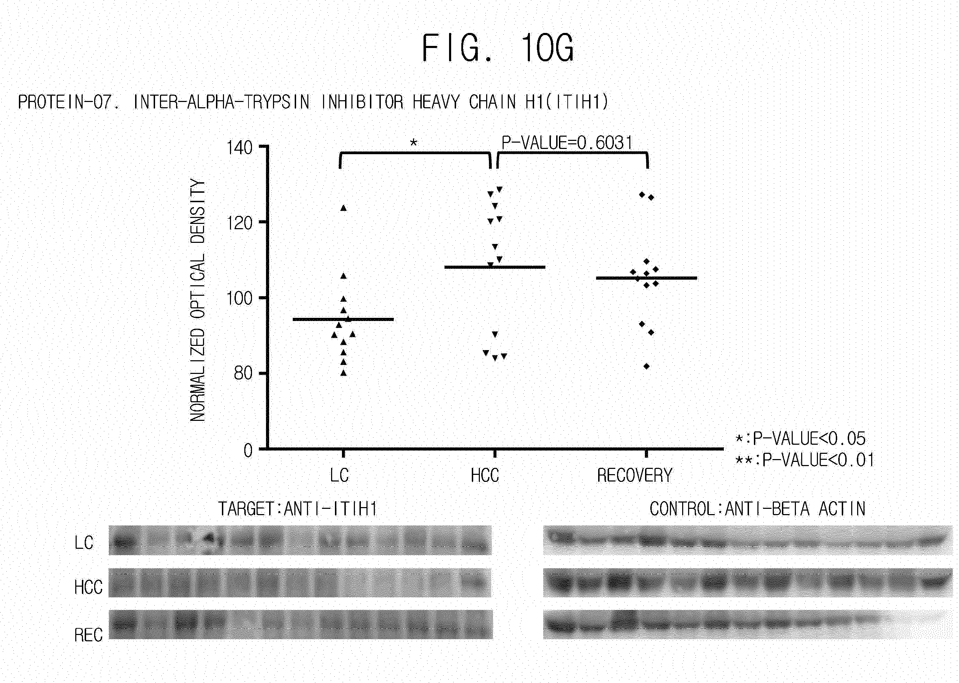

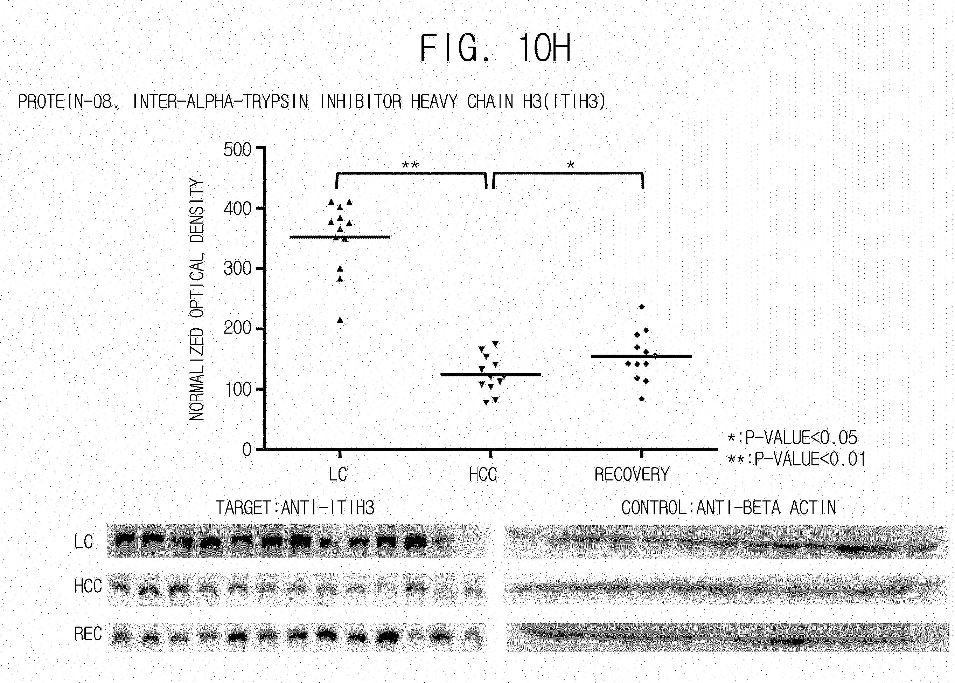

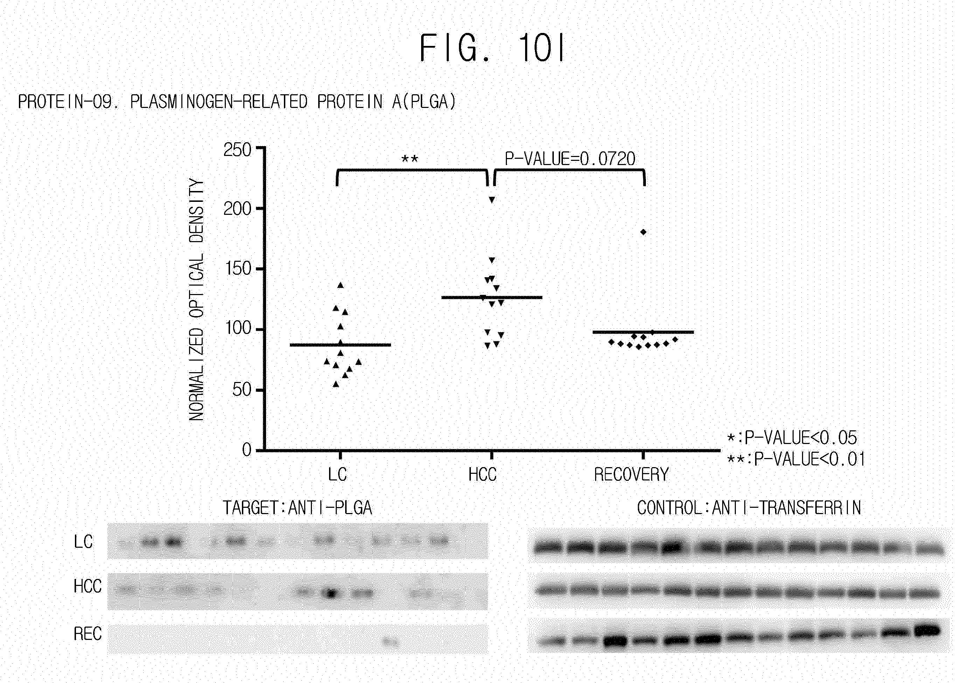

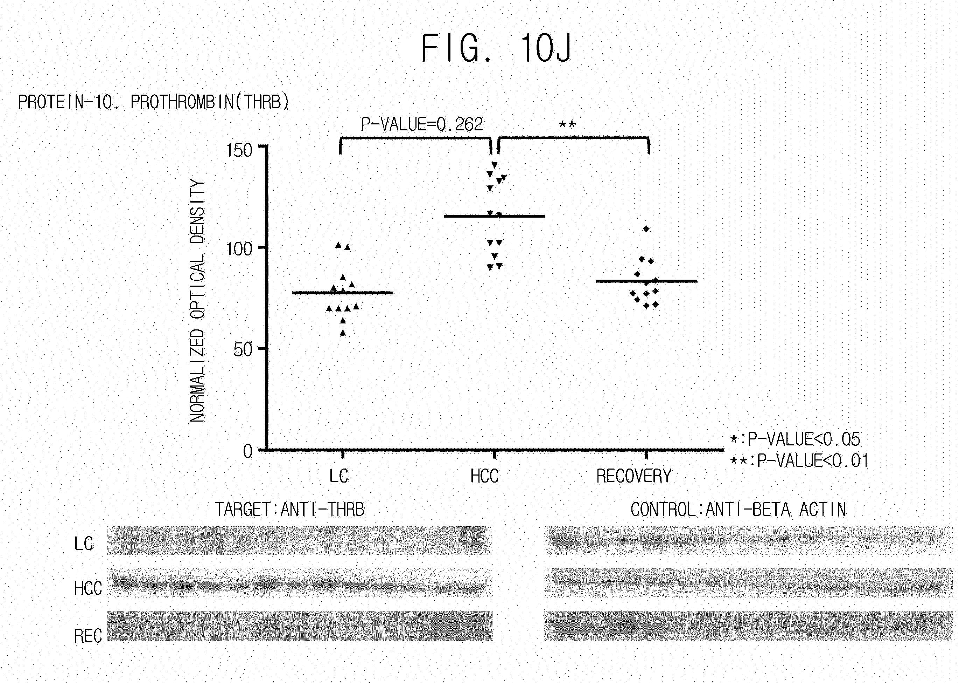

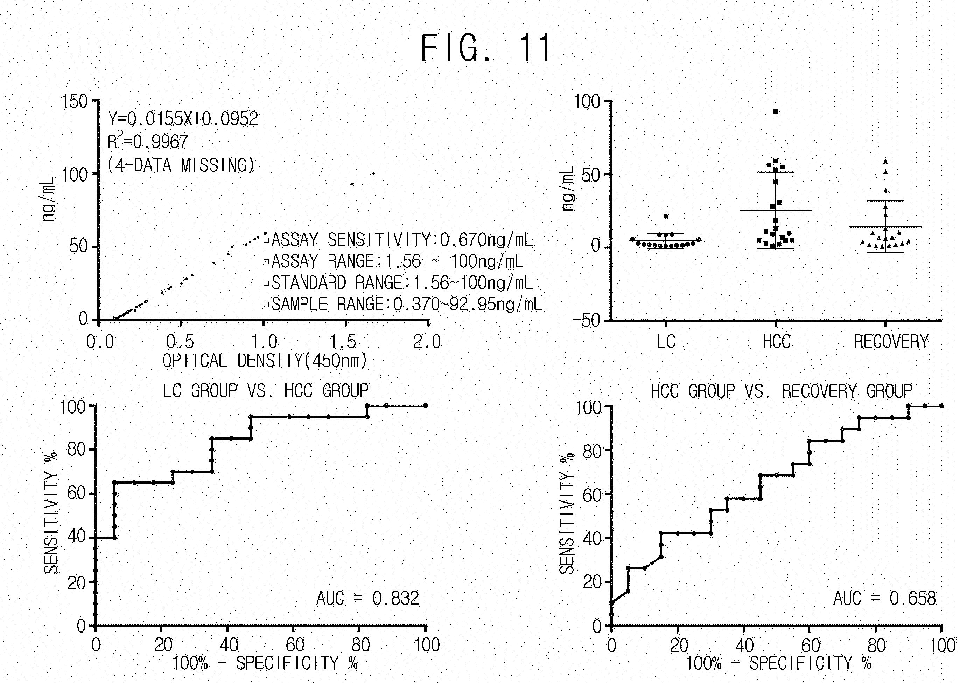

Fig. 10 illustrates results of western blotting for 10 targeted proteins which have same tendency of expression to verify biomarkers for early diagnosis, wherein Fig. 10A is for protein-01 and alpha-2-antiplasmin (A2AP); Fig. 10B is for protein-02 and AMBP; Fig. 10C is for protein-03 and alpha-fetoprotein (AFP); Fig. 10D is for protein-04 and cathepsin B (CATB); Fig. 10E is for protein-05 and alpha-2-HS-glycoprotein (FETUA); Fig. 10F is for protein-06 and fibronectin (FINC); Fig. 10G is for protein-07 and Inter-alpha-trypsin inhibitor heavy chain H1 (ITIH1); Fig. 10H is for protein-08 and Inter-alpha-trypsin inhibitor heavy chain H3 (ITIH3); Fig. 10I is for protein-09 and Plasminogen-related protein A (PLGA); and Fig. 10J is for protein-10 and Prothrombin (THRB).

The present invention is based on findings about proteins/peptides useful as biomarkers for diagnosing hepatoma, which shows significant difference between groups, by sequentially screening and verifying amount of distinct protein/peptide expressions of samples before and after treatment of patients with cirrhosis and hepatoma by using MRM.

Accordingly, the present invention relates to a composition or kit for diagnosing or prognosing hepatoma, including a detection reagent of at least one biomarker selected from a group consisting of insulin-like growth factor-binding protein complex acid labile subunit (IGFALS); alpha-1-microglobulin/bikunin precursor (AMBP); carboxypeptidase B2 (CPB2); cholinesterase (CHLE); clusterin (CLUS); beta-Ala-His dipeptidase (CNDP1); complement component C6 (CO6); carboxypeptidase N subunit 2 (CPN2); coagulation factor XI (FA11); fibronectin (FINC); hepatocyte growth factor activator (HGFA); insulin-like growth factor-binding protein 3 (IBP3); insulin-like growth factor II (IGF2); inter-alpha-trypsin inhibitor heavy chain H1 (ITIH1); kallistatin (KAIN); plasma kallikrein (KLKB1); phosphatidylcholine-sterol acyltransferase (LCAT); plasminogen-related protein A (PLGA); plasminogen (PLMN); prothrombin (THRB); vitamin D binding protein (VTDB); and vitronectin (VTNC).

Herein, hepatoma or hepatocellular carcinoma (HCC) refers to a primary malignant tumor occurring in liver tissue itself of patients with risk factors such as alcohol abuse, viral hepatitis, and metabolic liver diseases. Hepatoma causes bleeding and necrosis as well as blood vessel invasion into a hepatic portal system because there is no fibrous stroma, and leads to liver laceration and hemoperitoneum in severe cases. As chronic inflammation occurring in a liver, the hepatoma is distinguished from cirrhosis or fibrosis which makes liver tissues turn into tissues with regenerative nodules, and then fibrous tissues and finally shows symptoms such as liver cell damage, scar, impaired liver function, ascites, hemorrhagic disorders, and hematic comma. Patients with hepatoma generally have both hepatitis or liver inflammation and cirrhosis. While hepatitis disappears by medicines in most of patients, approximately 90% of patients with cirrhosis are diagnosed with hepatoma. Therefore, it is important to distinguish cirrhosis from hepatoma.

Herein, a term "diagnosis" includes determining susceptibility to a specific disease or illness of a test subject, determining whether or not the specific disease or illness is present, determining a transitional cancer condition, a cancer phase, or a cancer stage of the subject with the specific disease or illness, determining responsiveness of the cancer to a therapy, or using therametrics, e.g., to monitor a state of the subject to provide information on therapeutic effects.

Herein, a term "prognosis" includes determining whether or not a patient recovers from hepatoma after diagnosis and treatment.

Herein, a term "a biomarker for diagnosis or a diagnostic marker, " is a substance with which a patient with hepatoma may be diagnosed separate from a person with healthy cells, a patient with cirrhosis, or a person who has been properly treated for hepatoma, and includes protein that shows change in concentration particularly in blood, polypeptide derived from the protein, a gene coding the protein, or its fragment in a sample derived from a patient with hepatoma compared to a sample from a control group.

The amount of the biomarker in accordance with the present invention shows increase in the blood of the patient with hepatoma compared to the sample of the control group, and its sequence, for example, may be searched with an ID described in Table 1 in UniProt DB (www.uniprot.org).

In accordance with the present invention, the biomarker may be used as a combination of one or more biomarkers, for example, a combination of two, three, four, or five of them. Also, conventional biomarkers, e.g., AFP, and/or a diagnostic method, e.g., liver ultrasonography may be used together with the biomarker. Those skilled in the art could select combinations of biomarkers that satisfy targeted sensitivity and specificity through a method such as biological sample analysis and/or logistic regression analysis of subjects including normal persons and patients as described in accordance with one example embodiment of the present invention.

In accordance with one example embodiment of the present invention, at least one of ALS, CBPB2, CLUS, CNDP1, CPN2, FA11, FINC, or HGFA, particularly, is used and the biomarkers showed different expression between the control group and patients with hepatoma with high AUC values from both first and second batches of samples as described in accordance with one example embodiment of the present invention.

In accordance with another example embodiment of the present invention, biomarkers are used with FINC only, or in a combination of THRB and FINC, or in a combination of ALS, ITIH1, THRB, and FINC.

In accordance with another example embodiment of the present invention, a biomarker that determines whether or not patients recovered from hepatoma after treatment is selected from a group consisting of THRB, FINC, ITIH1, IBP3, PLMN, CBPB2, and PLGA, and although the above-described biomarker or the above-described combination of the biomarkers have particularly high AUC values, combinations of other biomarkers would not be excluded.

In accordance with another example embodiment of the present invention, the at least one biomarker may be used in a combination with AFP.

The biomarker in the present invention may be detected at levels of a nucleic acid, particularly, at a level of presence of protein and/or mRNA, an expression level itself thereof, or change or difference in the expression level thereof, through qualitative or quantitative analysis.

Herein, a term "detection" includes detection of presence or non-presence and of an expression level, quantitative and/or qualitative analysis and these methods have been disclosed in this industry and those skilled in the art could select an appropriate method to execute the present invention.

Detection of the biomarker in accordance with the present invention may be based on its functional and/or antigenic characteristics.

In accordance with one example embodiment of the present invention, the biomarker may be detected by using a nucleic acid that activates the biomarker, detects its function, or codes protein, particularly by using a substance specifically interacts at a level of mRNA and/or protein.

In accordance with another example embodiment, the detection of the biomarker in accordance with the present invention may be performed through detection of a peptide derived from a biomarker protein, for example, a peptide corresponding to each biomarker described in Tables 1-1 and 1-2. One or more peptides may be used for one protein.

A detection reagent included in a composition in accordance with the present invention is a reagent that may detect the biomarker in accordance with the present invention in a variety of methods at a level of protein or a nucleic acid through quantitative or qualitative analysis.

For the quantitative and qualitative analysis of the biomarker in accordance with the present invention, a variety of methods for detecting the disclosed protein or nucleic acid qualitatively or quantitatively.

As a qualitative or quantitative detection method at a level of protein, a method, e.g., that uses western blot, enzyme-linked immunosorbent assay (ELISA), radioimmunoassay (RIA), immunodiffusion, immunoelectrophoresis, immunohistochemistry (IHC), immunoprecipitation assays, complement fixation assays, binding with indexed antibodies in solutions and suspensions, mass spectrometry (MS), or protein array using antibodies may be used.

As a qualitative or quantitative detection method at a level of nucleic acid, a nucleic acid transcription and amplification method, an eTag system, an indexed-bead system, an array system such as a nucleic acid array, or others may be used.

These methods have been disclosed. For example, chip-based capillary electrophoresis (CE): Colyer et al. 1997. J Chromatogr A. 781(1-2):271-6, mass spectroscopy: Petricoin et al. 2002. Lancet 359: 572-77, eTag systems: Chan-Hui et al. 2004. Clinical Immunology 111:162-174, microparticle-enhanced nephelometric immunoassay: Montagne et al. 1992. Eur J Clin Chem Clin Biochem. 30:217-22, etc. may be referred to.

In accordance with one example embodiment of the present invention, a biomarker may be detected by using mass spectrometry (MS), and may be analyzed by separating proteins or peptides from a sample, e.g., just as in a method described in an example embodiment. For example, Kim, et al. 2010 J Proteome Res. 9: 689-99; Anderson, L et al. 2006. Mol Cell Proteomics 5: 573-88 may be referred to. In one example embodiment, for example, multiple reaction monitoring (MRM) by utilizing a triple quadrupole LC-MS/MS, QTRAP, etc. is used. The MRM is a method for quantitative measuring of a substance, such as a small amount of biomarker present in biological samples, in multiple ways, in which precusor ions or parent ions among ion fragments are created from an ionization source selectively and sent to a collision cell by using a first mass filter Q1. After that, the precursor ions in the collision cell collide with internal collision gas and splits into product ions or daughter ions, which are sent to a second mass filter Q2. Only characteristic ions are sent to a detecting unit. This is an analytic method with high selectivity and high sensitivity that may detect information on required ingredients only. For instance, Gillette et al., 2013, Nature Methods 10:28-34 may be referred to.

In accordance with another example embodiment of the present invention, a linkage agent specifically binding with individual proteins or mRNA derived from genes coding the protein, or an array including the linkage agent is used.

In accordance with still another example embodiment of the present invention, a sandwich-type immunoassay method such as ELISA, or RIA may be used. For example, the method may add a biological sample to a first antibody combined with a bead, a film, a slide, or a microtiter plate made with a solid substrate, e.g., glass, plastic such as polystyrene, polysaccharide, nylon, or nitrocellulose and then qualitatively or quantitatively detect protein labelled with a directly or indirectly detectable biomarker substance, e.g., a radioactive substance such as ³H or ¹²⁵I, fluorescent substance, chemiluminescent substance, hapten, biotin, digoxigenin, etc. or labelled with a combination of a conjugated antibody with an enzyme such as horseradish peroxidase (HRP), alkaline phosphatase (ALP), malate dehydrogenase capable of colorimetry or luminescence through an reaction with a substrate.

In accordance with another example embodiment of the present invention, immunoelectrophoresis such as Ouchterlony plate, western blot, crossed IE, rocket IE, fused rocket IE, or affinity IE, wherein a biomarker may easily detect through antigen-antibody binding, may be used. The immunoassay and the immunohistochemistry are described in Enzyme Immunoassay, E. T. Maggio, ed., CRC Press, Boca Raton, Florida, 1980; Gaastra, W., Enzyme-linked immunosorbent assay(ELISA), in Methods in Molecular Biology, Vol. 1, Walker, J.M. ed., Humana Press, NJ, 1984, etc. It is possible to diagnose whether or not a disease occurs by analyzing a final signal strength by the aforementioned immunoassay process, i.e., by performing comparison between the signal and that of the normal sample.

Reagents or substances used in these methods have been disclosed. For example, antibody, substrate, nucleic acid, or peptide aptamers specifically binding with the biomarker, or receptors, ligands, or cofactors, etc. that specifically interact with the biomarker may be used. The reagents or substances that are specifically interacting or binding with the biomarker in accordance with the present invention may be used together with a chip or nanoparticles.

The biomarker in accordance with the present invention may be qualitatively or quantitatively detected by using a variety of conventional methods at a level of nucleic acid, especially mRNA.

As a qualitative or quantitative detection method at a level of nucleic acid, for example, for detection at a level of mRNA, or detection of an expression level or a pattern, a method using reverse transaction polymerase chain reaction (RT-PCR)/PCR, competitive RT-PCR, real-time RT-PCR, Nuclease protection assay, NPA, such as RNase, S1 nuclease analysis, *in situ* hybridization, a DNA microarray, a chip, or northern blot, etc. may be used. This analysis has been disclosed and may be performed with a kit bought from a marketplace and those skilled in the art could select an appropriate one to implement the present invention. For example, the northern blot may identify the size of transcriptome in a cell and utilize a variety of probes. The NPA is useful for a multi-marker analysis, and *in situ* hybridization can easily identify the location of transcriptome such as mRNA in a cell or tissue while the RT-PCR is useful for detection of small amount of a sample. Besides, a linkage agent specifically binding with a nucleic acid such as mRNA or cRNA derived from genes coding biomarker protein in accordance with the present invention, or an array including the linkage agent may be used.

The reagent or substance used in the method for detecting biomarkers at the level of nucleic acid has been disclosed. A detection reagent in a method for measuring whether or not mRNA exists and the amount with the RT-PCR, for example, includes polymerase, a probe and/or a pair of primers specific to mRNA of the biomarker in the present invention. The term "primer" or "probe" means nucleic acid sequence with a free 3' hydroxyl group that may complementarily bind with a template and allow RTase or DNA polymerase to replicate the template. The detection reagent used in the present invention may be labelled with a chromatic, luminescent, or fluorescent substance as explained to detect a signal. In accordance with one example embodiment of the present invention, the northern blot or the RT-PCR is used to detect mRNA. The latter is a method for separating RNA, particularly mRNA in a sample, synthesizing cDNA from the RNA, and detecting a specific gene in the sample by using a specific primer or a combination of a primer and probe, that can determine presence or non-presence, or an expression level of the specific gene. This method is described, for example, in Han, H. et al, 2002. Cancer Res. 62: 2890-6.

A detection reagent included in a composition in accordance with the present invention may be labelled directly or indirectly in a shape of sandwich to detect depending on detailed methods used for detection. In case of the direct labeling method, serum samples used in an array, etc. may be labelled by fluorescent biomarkers such as Cy3 and Cy5. In case of the sandwich method, serum samples which are not labelled are first reacted and combined with an array with the detection reagent attached and then a biomarker protein is detected by being combined with a labelled antibody for detection. The sandwich method may perform detection at the level of pg/mL by being capable of increasing sensitivity and specificity. Other radioactive materials, chromatic substances, magnetic particles and high density electronic particles, etc. may be used as biomarker substances. For spectrofluorometry, a scanning confocal microscope may be used. For example, it may be purchased from Affymetrix, Inc. or Agilent Technologies, Inc., etc.

The composition of the present invention may include at least one additional ingredient necessary for linkage analysis. For example, a binding buffer, a reagent necessary to prepare a sample, a syringe for blood collection, or a negative and/or positive control group may be additionally included.

The composition of the present invention that includes a variety of detection reagents as explained above may be provided for ELISA, dipstick rapid kit analysis, MRM analysis, microarray, gene amplification, or immunoassay, etc. depending on forms of analysis and an appropriate detection reagent fit for analysis may be selected.

In accordance with one example embodiment of the present invention, the ELISA assay or the dipstick rapid kit is used, in which case, an antibody that recognizes at least one of biomarkers in accordance with the present invention may be provided, being attached to a substrate, for example, a well in a multiwell plate, the surface of a glass slide, or nitrocellulose. The dipstick rapid kit is a technology widely used in the field of point of care test (POCT) which is a chromatic method for detecting a biomarker, which shows color when a sample binds with an antibody in a substrate, by making a sample such as serum move on a substrate like nitrocellulose combined with at least one of antibodies that recognize the biomarker in accordance with the present invention due to capillarity when the sample contacts the substrate, e.g., when one end of a dipstick is dipped into the sample.

In accordance with another example embodiment of the present invention, a MRM kit based on peptides is provided and the MRM is the same as explained above. The MRM, which uses peptides selectively recognizing specific proteins, may detect a biomarker more stably from a biological sample compared to the existing methods using antibodies sensitive to the environment such as temperature, humidity, etc. For example, peptides as stated in Tables 1-1 and 1-2 herein may be used. For one biomarker, at least one peptide may be used. For example, peptides as a one-letter notation for amino acid sequences, which correspond to protein include Prothrombin (THRB) - HQDFNSAVQLVENFCR, SGIECQLWR; Fibronectin (FINC)-WCGTTQNYDADQK, GEWTCIAYSQLR, HTSVQTTSSGSGPFTDVR; ITIH1-EVAFDLEIPK, LDAQASFLPK; IBP3-ALAQCAPPPAVCAELVR; PLMN-LSSPAVITDK, EAQLPVIENK; CBPB2-DTGTYGFLLPER, YPLYVLK; and PLGA-DVVLFEK.

In accordance with another example embodiment of the present invention, an array including a microarray or a chip method may be provided. A detection reagent may be attached to the surface of a substrate such as glass or nitrocellulose. For the array technique, for example, Schena et al., 1996, Proc Natl Acad Sci USA. 93 (20) :10614-9; Schena et al., 1995, Science 270(5235):467-70; and U.S. Pat. Nos. 5,599,695, 5,556,752 or 5,631,734 may be referred to. The detection reagent that may be attached to an array may include an antibody, an antibody fragment, an aptamer, an avidity multimer, or peptidomimetics capable of being specifically binding with one protein.

The present invention relates to an analysis kit or system for diagnosing or prognosing hepatoma including a biomarker detection reagent. A detection reagent and a method using the reagent are as explained above. The reagent which may detect the biomarker may exist, being dispensed individually, in a container with partitions. In this meaning, the present invention relates to a device/kit that keeps the biomarker detection reagents partitioned. In addition, the kit may additionally include a user manual.

In accordance with another example embodiment, to provide information required to diagnose or prognose hepatoma, the present invention relates to a method for detecting a biomarker for diagnosis of hepatoma, including a step of detecting presence and/or concentration of a nucleic acid and/or protein of at least one biomarker selected from a group consisting of Insulin-like growth factor-binding protein complex acid labile subunit (ALS), Alpha-1-microglobulin/bikunin precursor (AMBP), Carboxypeptidase B2 (CPB2), Cholinesterase (CHLE), Clusterin (CLUS), Beta-Ala-His dipeptidase (CNDP1), Complement component C6 (CO6), Carboxypeptidase N subunit 2 (CPN2), Coagulation factor XI (FA11), Fibronectin (FINC), Hepatocyte growth factor activator (HGFA), Insulin-like growth factor-binding protein 3 (IBP3), Insulin-like growth factor II (IGF2), inter-alpha-trypsin inhibitor heavy chain H1 (ITIH1), Kallistatin (KAIN), Plasma kallikrein (KLKB1), Phosphatidylcholine-sterol acyltransferase (LCAT), Plasminogen-related protein A (PLGA), Plasminogen (PLMN), Prothrombin (THRB), Vitamin D binding protein (VTDB), and Vitronectin (VTNC) from a biological sample derived from a subject.

The method in accordance with the present invention further includes steps of comparing a result from detecting the level or the presence of the nucleic acid or the protein with a result corresponding to a biomarker of a sample of a control group, and determining as the hepatoma if there is any change in the concentration or any change in a state of the presence of the nucleic acid or the protein of the sample of the subject in comparison with the sample of the control group.

In the method in accordance with the present invention, samples derived from normal persons or patients that completely recovered from hepatitis or cirrhosis, or patients with cirrhosis that completely recovered from hepatoma may be used as samples available from a control group to diagnose patients with hepatoma. In comparison with the patients in the control group, an expression level of the biomarker in accordance with the present invention is increased in the patients with hepatoma.

As the biological sample used in the method in accordance with the present invention, whole blood, serum, or plasma is used.

In comparison with the sample derived from the normal persons as well as that of patients with cirrhosis, the expression level of the biomarker in accordance with the present invention increases in the patients with hepatoma.

Because hepatoma diagnosis of the method in accordance with the present invention is capable of distinguishing patients with cirrhosis and those with hepatoma, the method is more advantageous in diagnosing patients whose cirrhosis is developing into hepatoma, particularly, in early diagnosis. In this case, a sample derived from patients with cirrhosis as a control group may be included. The samples derived from the patients with cirrhosis include the samples from patients with cirrhosis who completely recovered from hepatoma. Besides, it is also favorable in determining patients who recovered from hepatoma after treatment. In this case, the samples of hepatoma derived from the very patients may be used as a control group.

Biological samples in the present invention refer to substances or mixtures of substances including at least one ingredient where a biomarker is detectable. They include organisms which include body fluids, particularly, whole blood, plasma, serum, or urine but they are not limited to these.

In accordance with one example embodiment of the present invention, at least one biomarker, particularly, from ALS, CBPB2, CLUS, CNDP1, CPN2, FA11, FINC, and HGFA is used and the biomarker showed different expression between the control group and hepatoma patients with high AUC values from both first and second batches of samples as described in accordance with one example embodiment of the present invention.

In accordance with another example embodiment of the present invention, the biomarker for determining whether recovery is achieved after treatment may be selected from a group consisting of THRB, FINC, ITIH1, IBP3, PLMN, CBPB2, and PLGA.

In accordance with another example embodiment of the present invention, FINC only, a combination of THRB and FINC, or a combination of ALS, ITIH1, THRB, and FINC are used as biomarkers.

In accordance with another example embodiment of the present invention, at least one biomarker may be used in a combination with AFP.

A control group or a reference group, a method for detecting a biomarker, a reagent used for it, and a method for analyzing data to determine used in this method may refer to the aforementioned above and the followings below.

The method in accordance with the present invention may be performed by using a method as stated above or a method using a detection reagent. In particular, it may be executed in a way of a protein or nuclear acid microarray analysis, nucleic acid amplification (NAT), antibody-antigen reaction, or mass spectrometry (MS).

The method in accordance with the present invention is targeting mammals, particularly, human beings. The human subjects include those who are suspected of having HCC, patients with hepatitis and cirrhosis, or those who are not suspected of having such diseases but need to get tested for HCC. In accordance with one example embodiment of the present invention, to determine whether or not a subject has HCC with liver disease-related symptoms such as abdominal pain, liver hypertrophy, hydrops abdominis, jaundice, muscular atrophy, hepatitis like HCV infection, or esophageal varix, the method may be performed on subjects who have different symptoms. In accordance with another example embodiment of the present invention, they are subjects who are normal persons in appearances and do not show HCC symptoms.

In accordance with another example embodiment of the present invention, the subjects are those who are not diagnosed with HCC based on plasma AFP concentration because they have low or normal plasma AFP concentration. In this case, the AFP serum concentration may range from approximately 0µg/l (non-detected) to 20µg/l, e.g., from 0µg/l (non-detected) to 5µg/l, from 5µg/l to 10µg/l, from 10µg/l to 15µg/l, or from 15µg/l to 20µg/l.

When a combination of biomarkers including at least two by using the aforementioned method is used, a data set including a profile, i.e., quantitative information related to biomarker protein expression among samples may be created.

After the profile is acquired by using the biomarkers, the method determines whether or not HCC is detected from the samples of the subjects through comparison with the result of a reference group or a control group. The control group or the reference group may be samples from normal persons or patients who recovered from HCC as a negative control group, and those derived from patients diagnosed with HCC, cirrhosis, and hepatitis as a positive control group according to a method beside the biomarkers in accordance with the present invention.

In accordance with one example embodiment of the present invention, samples derived from normal persons and samples derived from patients treated with HCC after being diagnosed are used as a control group or a reference group, thereby being compared with acquired profiles.

For comparison of biomarker profiles between the control group and the experimental group using samples, the disclosed method may be used. For example, comparison of digital images of expression profiles, comparison using database for expression data, or methods described in U.S. Patent 6,308,170 and 6,228,575 may be referred to.

The profiles acquired through the detection of biomarkers in accordance with the present invention may be processed by using the disclosed method for analyzing data. For example, a nearest-neighbor classifier, partial-least squares, SVM, AdaBoost and clustering-based classification may be used. For example, literatures including Ben-Dor et al (2007, J. Comput. Biol. 7: 559-83), Nguyen et al (2002, Bioinformatics 18:39-50), Wang et al (2003, BMC Bioinformatics 4:60), Liu et al (2001, Genome Inform. Ser. Workshop Genome Inform.12:14-23), Yeang et al (2001, Bioinformatics 17 Suppl 1:S316-22), and Xiong (2000, Biotechniques 29(6):1264-8, 1270), etc. may be referred to.

To determine that the result of the detection through the biomarker in the present invention is significant in determining HCC, a variety of statistical methods may be used. In accordance with one example embodiment of the present invention, a logic regression method as one of statistical methods is used and Ruczinski, 2003, Journal of Computational and Graphical Statistics 12:475-512 may be referred to. The method is similar to a CART method that suggests a binary tree as a classifier but a Boolean operator related to characteristics, as a more general operator compared to an "and" operator created by the CART, is used for each node. Nearest shrunken centroids (Tibshirani. 2002 PNAS. 99:6567-72), random forests (Breiman. 2001. Machine Learning 45:5-32, and MART (Hastie. 2001. The Elements of Statistical Learning, Springer) may be mentioned as examples of other analytical methods.

In accordance with one example embodiment, a significance level between a test substance and the control group may be determined to diagnose HCC through statistics. Raw data used for statistics are the values of individual biomarkers analyzed in double, triple, or multiple times.

The statistical analysis method is very useful in clinically significant determination through statistics of clinical and genetic data.

The method in accordance with the present invention may be used in determining degree of severity of HCC. For example, in comparison of the profiles of the positive control group and the negative control group, the severity may be evaluated as mild HCC, moderate HCC or severe HCC. In addition, a biomarker profile analysis for a certain HCC group may be performed and classification may be performed based on the profile result with a certain standard. Through early detection by this method, high-priced tests such as MRI may be avoided.

In accordance with another example embodiment of the present invention, the present method may be performed several times during a specific period, for example, over a year, and may be used to monitor development of changes in expression patterns. The increase or decrease of the expression depending on types of biomarkers may be related to the status of the HCC. In comparison with the previous test values of the same subjects or those of the control group, the values may be used to determine onset, development, deterioration, etc. of the HCC. Based on the change in HCC biomarkers with the lapse of time, preventive measures against the development of the HCC or the severe HCC may be taken. For a definitive diagnosis of the HCC, the biomarkers may be used as a supplementary means, together with an AFP test, an ultrasound scan, a computerized axial tomography (CT scan), or a magnetic resonance imaging (MRI).

To help the understanding of the present invention, example embodiments are proposed. However, the example embodiments below are provided to make the present invention understood more easily but the present invention is not limited to the following example embodiments:

### Example Embodiments

### Example embodiment 1. Information on clinical samples used in the present invention

The present experiment was performed under a protocol approved by the Institutional Review Board of Seoul National University, and received written consents of individual patients following thorough explanation. Clinical information is as follows:
To select a group of protein candidates, to be used for selection of a group of biomarker candidates, that showed expression differences in pooled samples of normal persons and patients with hepatoma, an initial study was conducted with samples of 60 normal persons of 41 men and 19 women and 60 patients with hepatoma of 42 men and 18 women. To analyze individual samples, a first sample group of 30 patients with cirrhosis, 30 patients with hepatoma before treatment, and 30 patients who recovered from hepatoma after treatment and a second sample group of 50 patients with cirrhosis, 50 patients with hepatoma before treatment, and 50 patients who recovered from hepatoma after treatment were used. The samples before and after hepatoma treatment were extracted from same patients. Only the samples of recovered patients who completely recovered from hepatoma without recurrence and metastasis were included for analysis, based on the serum samples collected before treatment and six-month follow-up data of the serum samples from the recovered patients after treatment.

### Example embodiment 2. Datamining for selecting a group of target candidates

A group of target candidates was selected as follows. A group of protein biomarker candidates for early diagnosis of hepatoma was secured by using the Liver Imaging Atlas database as the most comprehensive resources currently available in relation to liver diseases. There is a total of 50,265 proteins known to be related to liver diseases in the Liver Imaging Atlas database. To choose only proteins detectable in the blood among them, as a result of selecting proteins that are secreted, or have a possibility of being secreted into blood based on the Uniprot database, a total of 1,683 proteins was selected. To finally select proteins detectable with a mass spectrometer (MS), as a result of selecting proteins whose peptide MS/MS data exists by using four different peptide MS/MS library sources, i.e., NIST Ion-Trap, NIST Q-TOF, ISB human plasma, and home-made library, a total of 960 proteins was finally selected. To select a group of detectable target candidates, the following processes were conducted: To only select targets detectable with the MS, as a result of selecting peptides whose signals were properly detected by MRM analysis, 537 proteins and 1,316 peptides were selected from the pooled samples of 60 persons in the normal group and 60 in the HCC group.

### Example embodiment 3. Drawing a group of target candidates through MRM analysis

### Example embodiment 3-1. Preparation of serum samples

Serum samples used for the experiment were prepared as follows: Blood was collected by using a BD Vacutainer serum separation tube (BD, USA) and a Silica clot activator, 10mL, 16 X 100mm whose product number is 367820. In the collected blood, phenylmethylsulfonyl fluoride (PMSF) was added to finally reach 1.0mM. After the mixture was made by inverting approximately 10 times, a supernatant was collected by centrifugation at 3,000rpm for 10 minutes at the temperature of 4 °C. After the observation of the color, hemolyzed samples were not used.

The supernatant was separated by 100µL in each of 1.5mL Eppendorf tubes, and then it was placed in ice. The tubes were labelled with tracing codes corresponding to sample groups, e.g., NC-01, MC-01, PD-01, etc. After that, they were immediately kept at the temperature of -80 °C. The processes were performed in ice and were all finished within one hour after blood collection.

### Example embodiment 3-2. Serum protein depletion

To search for low-concentration biomarkers present in the blood, a depletion course was conducted first to remove six kinds of highly abundant proteins including albumin, IgG, IgA, haptoglobin, transferrin, and alpha-1-antitrypsin in the blood. By removing the six kinds of proteins, approximately 85% of total protein mass was removed from the blood and only the remaining 15% was used for analysis. By using the Multiple Affinity Removal System, MARS, of Agilent, USA, according to the method indicated by the maker, six proteins with the largest amounts in the blood were bound to the columns and then removed. The non-combining small amount of proteins were eluted and then used for analysis.

### Example embodiment 3-3. Peptidization of serum proteins

The serum samples obtained after the depletion as shown above were enriched with a 3K filter and protein concentration was quantified with a bicinchoninic acid assay (BCA). After being processed with final concentration of 6M urea/20mM DTT (Tris pH 8.0), the 100*µ*g serum samples were incubated for 60 minutes at the temperature of 37 °C. After being processed with 50mM final concentration IAA (Iodoacetamide), they were incubated at the room temperature for 30 minutes. 100mM Tris pH 8.0 was used to make the concentration of urea at least 0.6M or less. After a trypsin process to make the concentration ratio of trypsin and serum to be 1:50, the samples were incubated at the temperature of 37 °C for 16 hours. A formic acid solution was processed to have the final concentration of 5%, and then the following course of desalting was executed:

### Example embodiment 3-4. Desalting serum proteins

Activation was conducted by dripping three times 1 mL of 60% ACN/0.1% formic acid onto an OASIS column (Waters, USA). Equilibration was conducted by dripping five times 1 mL of 0.1% formic acid onto the OASIS column. The peptide samples were put in and then 1 mL of 0.1% formic acid was dripped five times for cleansing. Then 1 mL of 40% ACN/0.1% formic acid and 1 mL of 60% ACN/0.1% formic acid were used to elute the peptides. After being frozen for at least one hour at the temperature of -70 0 °C, the peptides were dried with Speed-vac. The dried peptides sample was resolved in 50 µℓ of Sol A buffer of 3% ACN/0.1% formic acid, and then was centrifuged at 15,000rpm for 60 minutes, among which only 40 *µ*ℓ was moved to a vial for analysis.

### Example embodiment 3-5. MRM analysis

For the MRM analysis, to select targets that have technical reproducibility for 537 proteins and 1,316 peptides selected as shown in Example Embodiment 2 and that show expression differences between the normal group and the HCC group, a set of pooled samples of 60 normal group persons divided into three sets of 20 and a set of pooled samples of 60 HCC group patients divided into three sets of 20, individually, were repeatedly analyzed with MRM three times per set.

Peptides and fragment ions for MRM analysis regarding individual target biomarker proteins were selected by using Skyline (http://proteome.gs.washington.edu/software/skyline). Skyline is an open source software for development and analysis of MRM (Stergachis AB, et al., 2011, Nat Methods 8: 1041-1043).

In summary, a full-length protein sequence, after being inputted in FASTA format into Skyline, was designed as peptides and a list of product ions was created and monitored with the MRM. The conditions for peptide filtering used to select transitions are as follows: with the maximum peptide length of 30 amino acids and the minimum length of 6 amino acids, but repeated arginine (Arg, R) or lysine (Lys, K) was not included. Even if methionine (Met, M) was included in the peptide, it was removed due to the possibility of transformation. Peptides with prolines next to arginine or lysine were not used but the peptides with histidine (His, H) were used even though the electric charge changes.

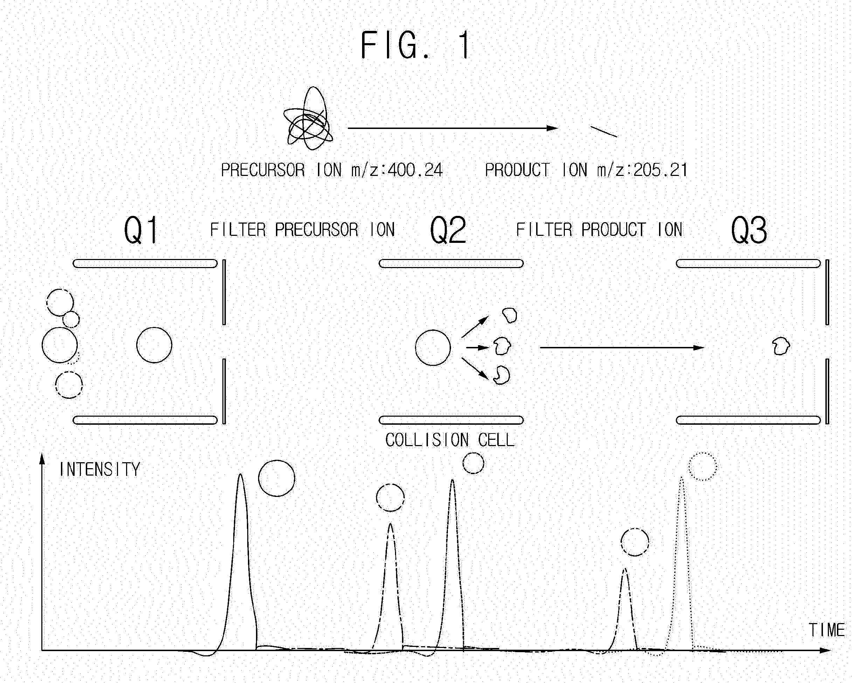

The peptides satisfying such conditions were used as transitions of a first quadruple Q1. The Q1 performed the role as a screening filter that may let pass only specific Q1 m/z. A precursor ion that passed the Q1 filter, was decomposed into a product ion with electric energy at a second quadruple collision cell by fragmentation. Only a specific product ion among those may pass a third quadruple Q3 that performs the role as a filter just like the first quadruple Q1. An ion that passed the third quadruple Q3 was converted to a digital signal by a detector and shown as a peak in a chromatogram. The area of the peak was analyzed and the analysis for relative quantification and absolute quantification was performed. A file including this information was put into the Analyst (AB SCIEX, USA) for MRM analysis, and was analyzed using non-scheduled MRM method. The result of the MRM was created as wiff and wiff.scan files, and these were converted into mzXML format by using mzWiff and inputted into Skyline for MRM data processing. Then the peak strength of the MRM transition was obtained. Fig. 1 schematically shows the course.

Upon all MRM analysis, for the purpose of ensuring quantification, with a specific peptide whose concentration has been already known, a process of normalizing making use of a peak area value of the peptide is conducted. The corresponding peptide is called an internal standard peptide, which is a peptide including amino acids having stable isotopes. In this study, when all samples were analyzed by using LNVENPK, a heavy-labeled peptide of beta-galactosidase (lacZ) derived from *E. coli,* which does not exist in human proteome, 5-fmol of the internal standard peptide was injected and a process of normalizing a peak area value of all target peptides from the MRM analysis making use of the peak area value of the internal standard peptide was conducted.

Via the semi-quantitative MRM analysis as shown above, to select only protein/peptide detectable with technical reproducibility by using the MRM technique, analysis was conducted repeatedly for three times per set and as a result of selecting only targets whose values of % CV are within 20 from at least one of two groups: a normal group or an HCC group, 347 proteins and 754 peptides were selected as reproducible targets.

Next, to select targets that showed expression difference between the normal group and the HCC group from the reproducibly detected targets, p-values and fold-change levels between the groups were confirmed. The analysis was conducted with the reproducible targets (protein 347, 754-peptides) as objects, judging by a normality test or Shapiro-Wilk test, using the independent T-test as a parametric method if normal distribution is followed with p-value > 0.05 and using the Mann-Whitney test as a non-parametric method if normal distribution is not followed with p-value ≤ 0.05. In case of the independent T-test, p-values were confirmed through Levene's test for assessment of the equality of variances by dividing into the case of equal variances with p-value > 0.05 and the case of unequal variances with p-value ≤ 0.05.

With the independent T-test and the Mann-Whitney test, the target protein/peptide was determined to show significance between the normal group and the HCC group with p-value ≤ 0.05.

In addition, a group of protein biomarker candidates which showed a pattern of increase or decrease of the fold change level between the normal group and the HCC group by more than 1.5 times was selected. Through this, it was found that there were 195 proteins and 443 peptides which show the expression differences between the normal group and the HCC group with the p-value equal to or less than 0.05 and that there were 191 proteins and 389 peptides with equal to or more than a 1.5-fold change. Through these, 227 proteins and 492 peptides were finally selected as a group of target candidates.

To confirm whether all of 227 proteins and 492 peptides selected were unique peptides in all human proteomes, the BlastP search program of NCBI was used. Through this, 15 proteins corresponding to 37 peptides which were not unique were excluded and 216 proteins and 460 peptides as a group of target candidates were finally selected as unique peptides that showed significant differences between the normal group and the HCC group.

**Example embodiment 4. Confirmation of whether the markers of the target candidate group are endogenous peptides**

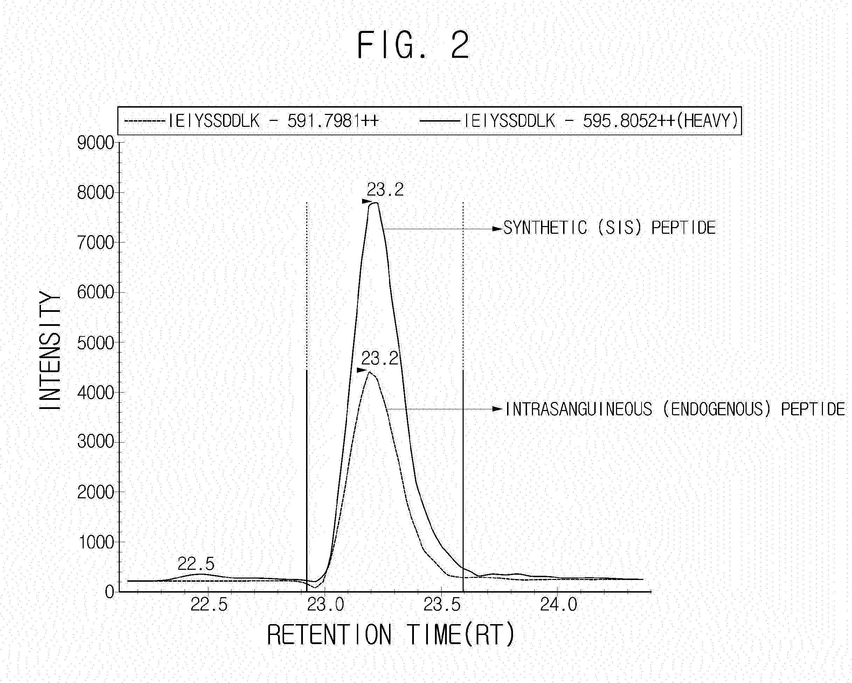

Whether the proteins and the peptides selected in the example embodiment 3 actually exist in the blood was confirmed as follows:
Whether or not the selected peptides are actually present in the blood was reconfirmed by using the pooled samples with 60 0 persons from the normal group and 60 persons from the HCC group by using stable-isotope labeled standard (SIS) peptides corresponding to 216 proteins. The SIS peptides are peptides including the arginine amino acids, i.e., Arg or R, or the lysine amino acids, i.e., Lys or K, at peptide C-terminal, whose ¹²C and ¹⁴N are replaced with ¹³C and ¹⁵N. Although it has the different mass value from an endogenous peptide present in the blood, it has same sequences and same peptide hydrophobicity, and therefore it is eluted onto the chromatogram at the same time (RT) as the peptide in the blood (See Fig. 2).

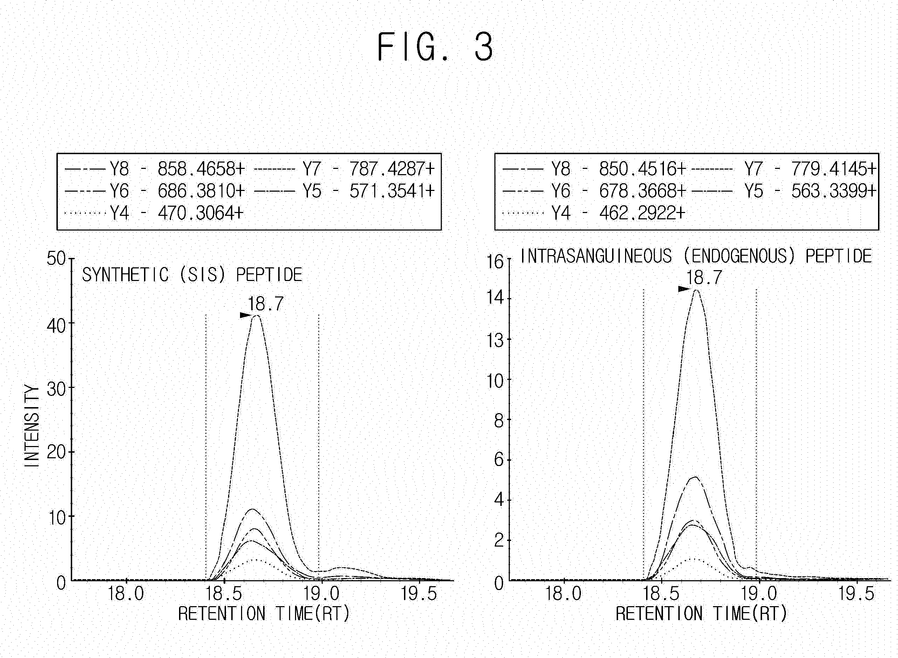

Upon the MRM analysis, to check whether there is any signal interference by a peptide other than the target peptide in the complex blood sample, intensity patterns of the product ion Q3 from the SIS peptide and the endogenous peptide in the blood were checked (See Fig. 3).

To determine whether there is any signal interference, an automated detection of inaccurate and imprecise transitions (AuDIT) program was used and the relative intensities of the product ions of the peptide in the blood and the SIS peptide were compared, with the P-value threshold of 0.05. Whether the detected peak area values of the peptide in the blood and the SIS peptide were consistent across repeated measures with the CV threshold of 0.2 was confirmed. Through this, 123 proteins and 231 peptides in the blood quantifiable without the signal interference were finally selected as endogenous target proteins and peptides.

### Example embodiment 5. Confirmation of endogenous peptide levels in the blood

As shown in the example embodiment 4, concentrations of the quantifiable endogenous targets including 123 proteins and 231 peptides present in the blood were confirmed. The MRM analysis of samples made by blending mixtures of 231 SIS peptides sequentially diluted at three points (20nM, 200nM, 2,000nM) and the pooled samples of 10µg from 60 persons in the normal group and 60 in the HCC group was performed. The signals of the endogenous peptides in the blood were confirmed together with those of the SIS peptides complementary to the endogenous peptides.

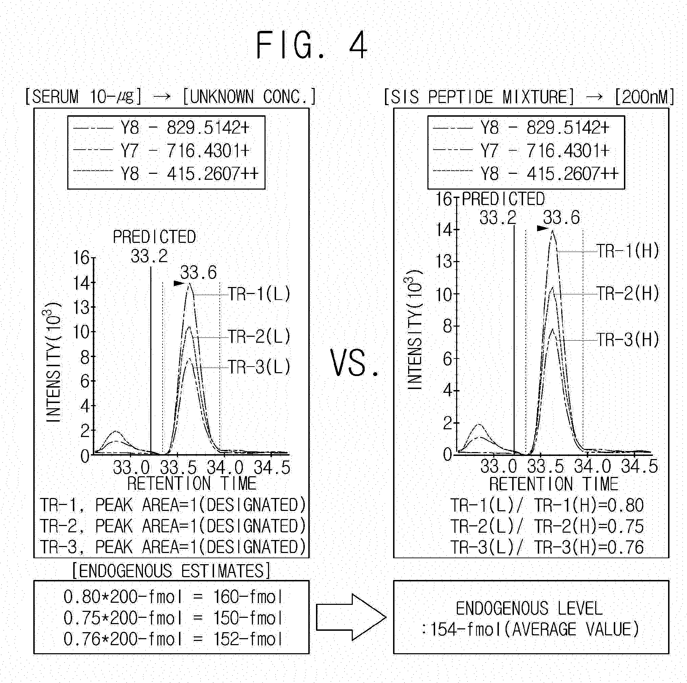

Peak area values of the peptides in the blood and those of complementary SIS peptides at 3 points, only for the product ions Q3 which do not have interference, were obtained and the relative ratio is calculated. The level of the target peptides in the blood was finally confirmed by multiplying the amount of the injected SIS peptides by the ratio (See Fig. 4).

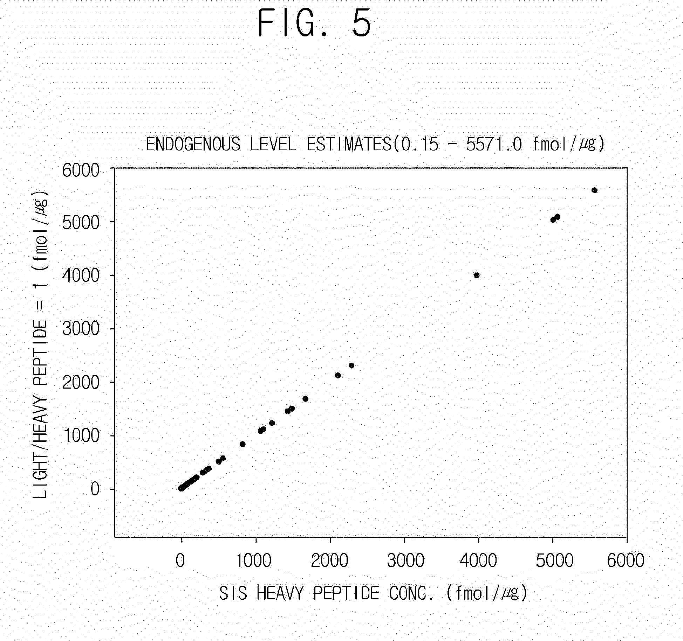

As a result of confirming the level of 231 peptides in the blood, immunoglobulin superfamily containing leucine-rich repeat protein, ISLR, was determined to be at the lowest concentration in the serum and the concentration was 0.15-fmol/ug and alpha-2-macroglobulin, A2MG, was determined to be at the highest concentration therein and the concentration was 5.57-pmol/µg (Dynamic range: 3.7×10⁴ order) (Fig. 5).

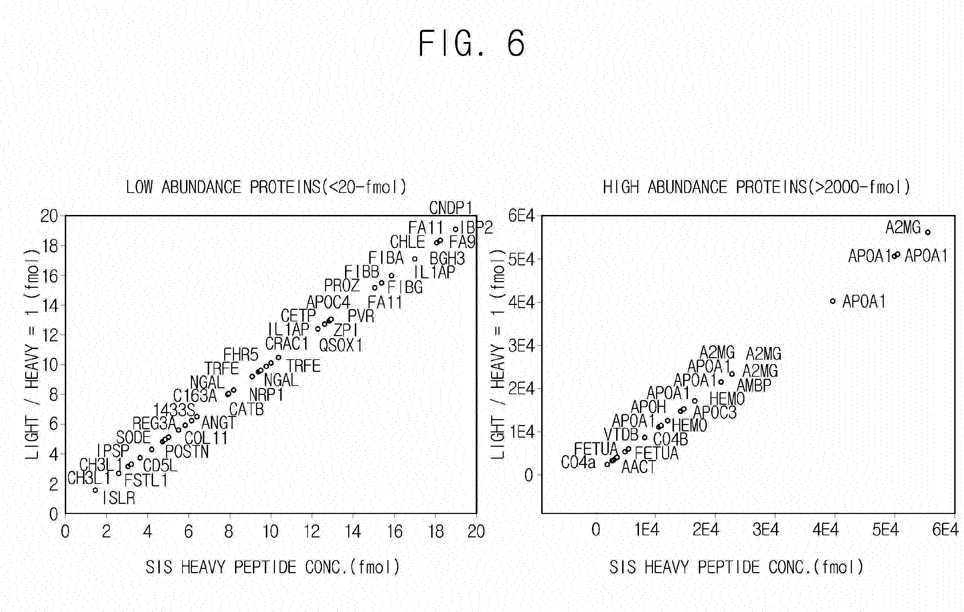

As a result of measuring the concentration of the endogenous peptides in the blood, it was confirmed that the low-abundance targets measured to have the blood concentration of less than 20-fmol/*µ*g were 34 proteins and 36 peptides; the middle-abundance targets measured to be at the range from 20-fmol/*µ*g to 2, 000-fmol/*µ*g were 93 proteins and 174 peptides; and the high-abundance targets measured to be more than 2,000-fmol/*µ*g were 11 proteins and 22 peptides (Fig. 6).

### Example embodiment 6. Drawing a group of biomarker candidates for early diagnosis of hepatoma through the application to individual samples

As approximately 90% of patients with cirrhosis are diagnosed with hepatoma, the patients with cirrhosis may be classified to be in a group of persons with high risks of hepatoma, but conventional serologic tests (AFP, PIVKA-II) used for diagnosis of hepatoma have weak diagnostic power with sensitivity of 30-40%. Accordingly, if it is possible to check in advance the amount of protein that shows difference between the patients with cirrhosis and the patients with hepatoma by using MRM, patients whose disease develops from cirrhosis to hepatoma may be diagnosed earlier.

The infusion concentration of the SIS peptides complementary to the endogenous peptides verified to be in the blood was decided. For low-abundance targets at the level in the blood < 20-fmol/*µ*g, SIS-peptides of constant amount 20-fmol/*µ*g were injected; for middle-abundance targets at the blood level of more than 20-fmol/µg and less than 2,000-fmol/µg, the amount of the SIS peptide same as the amount of peptides in the blood was injected; and for high-abundance targets at the blood level of more than 2,000-fmol/µg, SIS-peptides of constant amount 2,000-fmol/µg were injected.

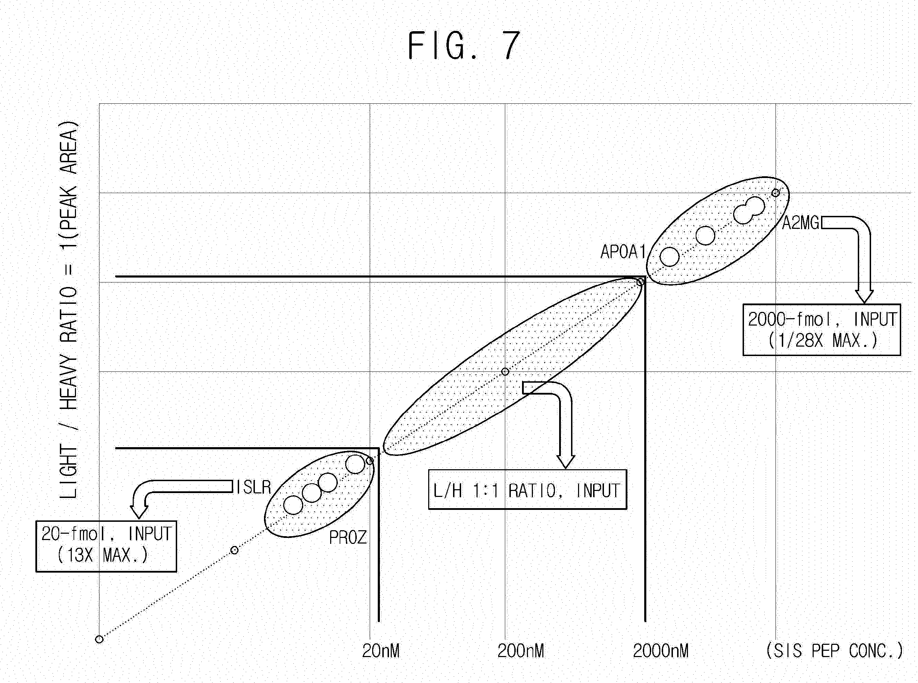

In case of the ISLR protein whose blood level was measured low, the SIS-peptides at 20-fmol were injected by up to 13 times as high as the level in the blood. In case of the A2MG protein whose level in the blood was the highest, the SIS-peptides at 2,000-fmol were diluted and injected by up to 1/28 time as low as the level in the blood (Fig. 7).

For the purpose of early diagnosis, with the finally verified group of target candidates which were 123 proteins and 231 peptides as subjects, samples of 30 patients with cirrhosis, 30 patients with hepatoma, and 30 patients who completely recovered from hepatoma as a first sample group were used; and biomarkers for diagnosis of hepatoma which showed differences among three groups, consisting of samples of 30 patients with cirrhosis, 30 patients with hepatoma, and 30 patients who completely recovered from hepatoma, independent of the first sample group, as a second sample group.

To prevent researchers from identifying patient groups, the researchers were blinded and then the MRM analysis was performed in a random sequence repeatedly three times per sample.

After peak area values of target peptides were obtained therethrough, those of peptides in the blood, and complementary SIS peptides were normalized, analysis was conducted using IBM SPSS statistics (version 21.0) and GraphPad (version 6.00).

### Example embodiment 7. Drawing a group of single biomarkers for early diagnosis of hepatoma

The early diagnosis test by using the conventional alpha-fetoprotein (AFP) cannot be used for early diagnosis of hepatoma because it is difficult to distinguish chronic liver diseases, e.g., chronic hepatitis and cirrhosis, from hepatoma. To suggest new biomarkers for early diagnosis to replace the test, comparisons between the LC group of patients with cirrhosis and the HCC group and between the HCC group and a recovery group which was a LC group of patients who completely recovered from hepatoma were conducted and analysis of the targets which showed the expression differences betwleen the LC group and the HCC group and between the HCC group and the recovery group was conducted. The result is described as shown in Table 1 below.

To identify a group of single biomarker candidates whose diagnostic power that may be used in the actual clinical area or hospitals for the purpose of early diagnosis of hepatoma is verified, i.e., to identify a group of single biomarker candidates whose characteristic as to whether a tendency of the same expression occurs in independent samples is verified, biomarkers with high AUC values, consistent across first samples as a training set and second samples as a test set, were found as a result of analysis. As the result of analysis with the training set and the test set, the both cases showed significant differences with P-value ≤ 0.05 between the LC group and the HCC group and between the HCC group and the recovery group, and targets with high diagnostic power with AUC-value ≥ 0.700 were finally identified as 23 proteins and 51 peptides derived from the proteins (Tables 1-1 and 1-2). Tables 1-1 and 1-2 represent target proteins and peptides with the AUC values equal to or greater than 0.700 after analyzing individual samples of the training set and the test set. The data inside the boxes with dotted lines in the tables below indicates the increase of the expression levels in comparison among groups and the data outside the four boxes indicates the decrease of the expression levels.

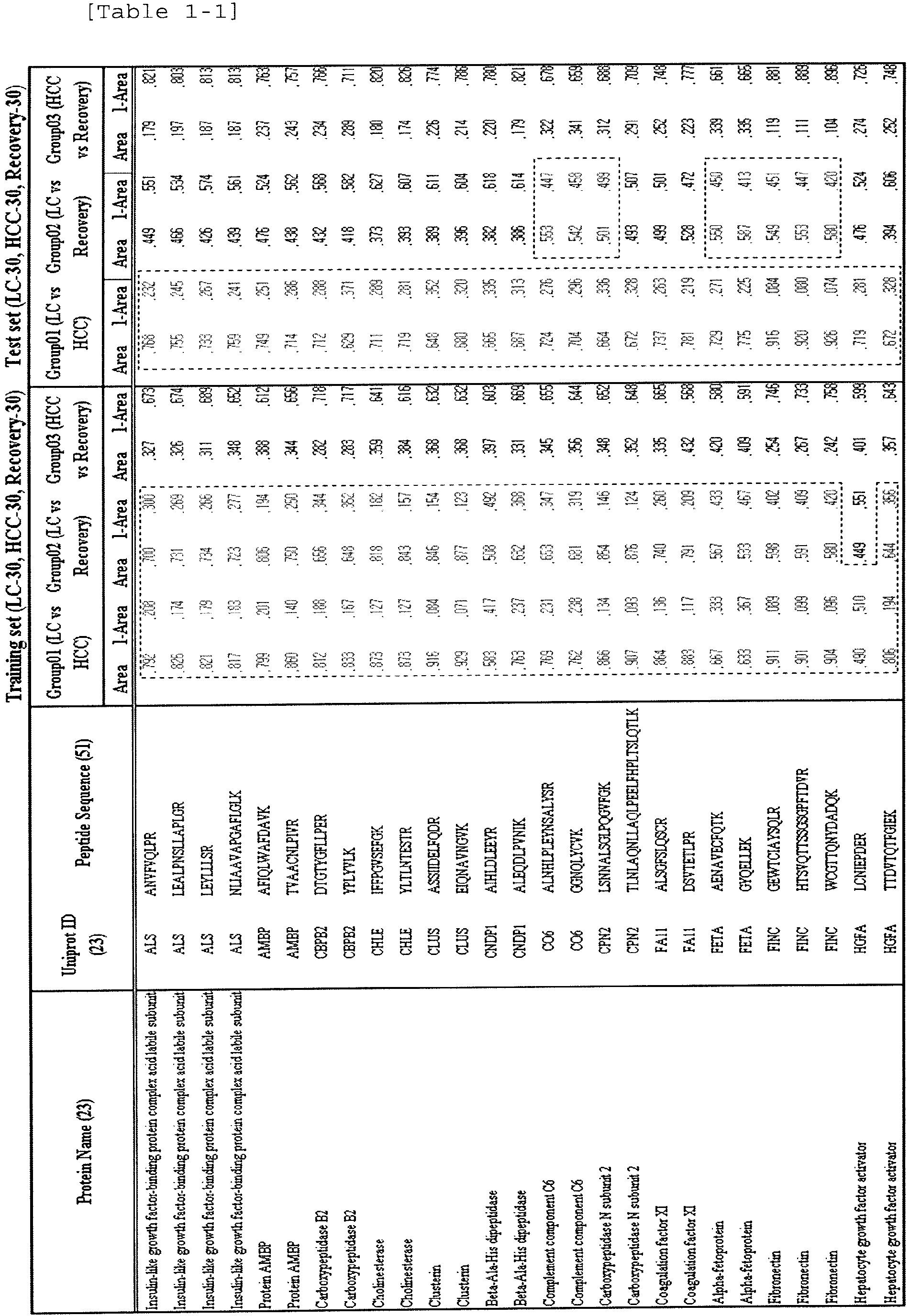

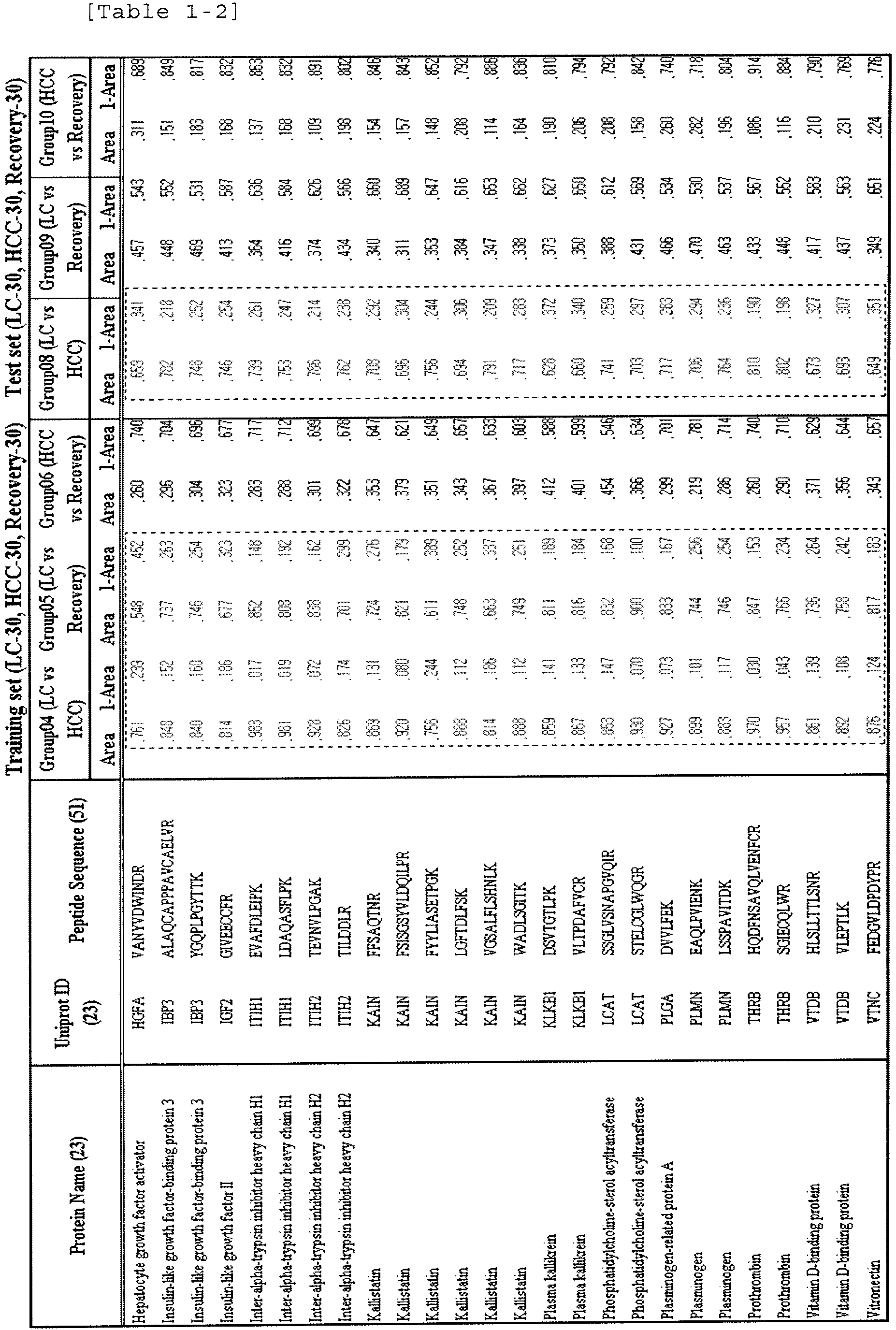

### Example embodiment 8. Drawing multi-biomarkers for diagnosis of hepatoma

As in the example embodiment 7, multi-protein biomarker panels were implemented and compared through multi-variate analysis (MA) of 23 proteins and 51 peptides that show consistent high diagnostic power between the LC group and the HCC group and between the HCC group and the recovery group from a result of analysis with first samples as a training set and second samples as a test set.

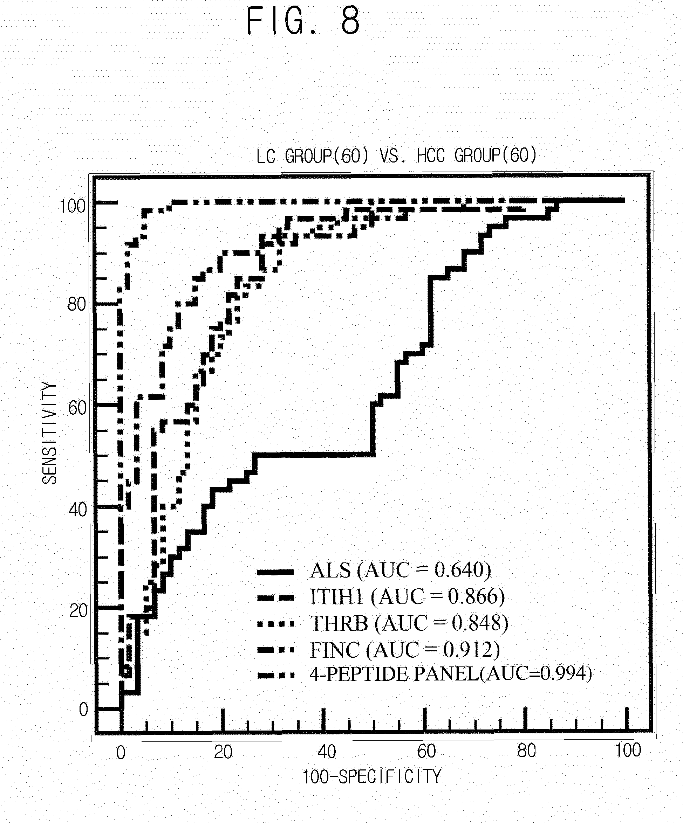

As a result of combining markers into one panel by using logistic regression, one of statistical methods for analysis, it was found that for comparison between the LC group and the HCC group, a panel of four peptides ALS + ITIH1 + THRB + FINC was a combination with the highest diagnostic power, and for comparison between the HCC group and the recovery group, a panel of two peptides THRB and FINC was a combination with the highest diagnostic power. More specifically, in comparison between the LC group and the HCC group, it was found that the confirmed panel of four peptides had the AUC value of 0.994, which means that it may diagnose 57 as patients with cirrhosis out of 60 patients with cirrhosis with the accuracy rate of 95.0%, and 58 as patients with hepatoma out of 60 patients with hepatoma with the accuracy rate of 96.7%. Therefore, it was found that the diagnostic accuracy of the 4-peptide or 4-protein biomarker panel was confirmed to be 95.8% (Fig. 8). Besides, it was confirmed that, if the remaining 19 proteins or 47 peptides are added to the 4 proteins or 4 peptides, it may be possible to make innumerable combinations of proteins with AUC value equal to or greater than 0.990.

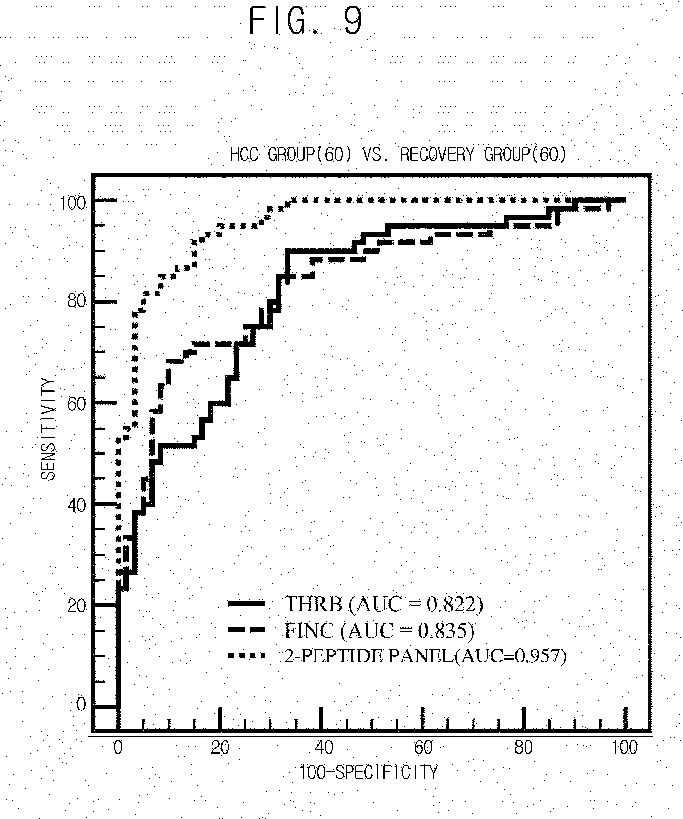

In comparison between the HCC group and the recovery group, the confirmed panel of two peptides was determined to have the AUC value of 0.957 (Fig. 9). This means that it may diagnose 53 as patients with hepatoma out of 60 patients with hepatoma with the accuracy rate of 88.3% and may diagnose 52 as patients with hepatoma out of 60 patients with cirrhosis who recovered from hepatoma after treatment with the accuracy rate of 86.7%. The diagnostic accuracy of the panel of two peptides was confirmed to be 87.5%. Besides, it was confirmed that, if the remaining 21 proteins or 49 peptides are added to the 4 proteins or 4 peptides, it may be possible to make innumerable combinations of proteins with the AUC value equal to or greater than 0.950.

### Example embodiment 9. Additional verification of biomarkers for diagnosis of hepatoma by using antibodies

Biomarkers in accordance with the present invention may be detected at a protein level and detection methods at the protein level may include MRM analysis and an analysis method using antibodies used in this example embodiment. The desired result in accordance with the present invention may be obtained with only one method, i.e., MRM analysis, or both of the two methods may be used for reconfirmation.

For reconfirmation through antibody analysis, among 23 proteins verified with MRM analysis at a peptide level as biomarkers for diagnosis of hepatoma, 7 proteins with top AUC values were selected and they were additionally analyzed by using the antibodies (Table 2).

Because an antibody recognizes an antigen consisting of some residues of the whole protein, a criterion for selecting antibody were that the peptidic part analyzed with the MRM be included in portions of the antibody-immunogen or be close to it. Antibodies which had a western blot result of plasma/serum and antibodies whose monoclonal antigens existed were first selected. The western blot using antibodies was performed for alpha-2-antiplasmin (A2AP) using mouse monoclonal, AMBP using rabbit monoclonal, alpha-fetoprotein (AFP) using mouse monoclonal, FETUA using rabbit monoclonal, fibronectin (FINC) using rabbit monoclonal, inter-alpha-trypsin inhibitor heavy chain H1 (ITIH1) using mouse polyclonal, inter-alpha-trypsin inhibitor heavy chain H3 (ITIH3) using goat polyclonal, plasminogen-related protein A (PLGA) using rabbit polyclonal, and prothrombin (THRB) using rabbit monoclonal (Santa Cruz Biotechnology, USA).

For this, samples of 12 persons per three groups including LC group, HCC group, and recovery group were selected, which were independent samples not used for first and second MRM analyses. The selected samples of the HCC group and the recovery group were at the Stage I, i.e., early hepatoma stage, and normalized with optical density, O.D., measured from the control group, i.e., a loading control, to calibrate variation between SDS-PAGE gels. For the control group, the mouse monoclonal antibodies for beta-actin and transferrin were used.

The result is illustrated in Fig. 10. The result shows that the biomarkers in accordance with the present invention are capable of being analyzed through a variety of methods for analysis of proteins.

For the purpose of applying and using one kind of the target protein FINC with the highest classifying capacity between groups in the western blot test by using the antibodies, ELISA analysis at the protein level in a native form was additionally conducted. To perform the ELISA analysis, samples of 20 persons per three groups including LC, HCC, and Recovery were selected and these were independent samples not used in the first and the second MRM analysis.

As a result of analyzing the target protein FINC by using an ELISA kit (Uscn Life Science Inc. Korea) as instructed by the kit maker, all the expression patterns between sample groups were identical to those of results from the MRM analysis and the western blot test. It was finally found that the AUC value was 0.832 in comparison between the group of patients with cirrhosis and the group of patients with hepatoma and that the AUC value was 0.658 in comparison between the group of patients with hepatoma and the group of patients who recovered from hepatoma (Table 2). The result represents that the biomarkers in accordance with the present invention may be developed and used as a variety of protein analysis kits such as MRM, western blotting, and ELISA.

**[Table 2] Information on ELISA analysis**

| • **ELISA analysis result** | | | | | | |
|---|---|---|---|---|---|---|
| **Protein Name** | **Uniprot ID** | **Assay Sensitivity** | **Assay Range** | **Standard Sample Range** | **Sample Range** | **Mssing Data** |
| Fibronection | FINC | 0.670 ng/mL | 1.56-100 ng/mL | 1.56-100 ng/mL | 0.370-92.95 | 4 |
| | | | | | | |

| **LC group vs. HCC group** | | | | | | |
|---|---|---|---|---|---|---|
| AUC Value | Cutoff Value | Sensitivity / Specificity | | | | |
| 0.832 | 8.95 ng/mL | 65.0% /94.1% | | | | |
| | | | | | | |

| **HCC group vs. Recovery group** | | | | | | |
|---|---|---|---|---|---|---|
| AUC Value | Cutoff Value | Sensitivity / Specificity | | | | |
| 0.658 | 4.44 ng/mL | 85.0% / 42.1% | | | | |

The biomarkers in accordance with the present invention may diagnose hepatoma with high specificity and high sensitivity. In particular, they may classify patients with hepatoma as well as patients with cirrhosis and patients with hepatoma. Therefore, the biomarkers may be used for prognosis of hepatoma treatment, as patients with cirrhosis who is developing hepatoma may be diagnosed in advance and patients with cirrhosis who completely recovered from hepatoma may be identified.

In addition, the biomarkers in accordance with the present invention are very useful in early diagnosis at home or in general practice with noninvasive tests by using blood and may bring an effect of saving medical costs at the state level because it is easy to diagnose hepatoma through simple blood tests upon health examination.

The biomarkers in accordance with the present invention may be developed into a form of diagnostic kit by using their antibodies, proteins, or peptides, for example, ELISA, a dipstick-typed rapid test kit, or an MRM kit based on peptides. In particular, they may be commercialized with a method for testing peptides with MRM instead of immunoassays in the conventional clinical areas of hospitals, and furthermore, they may be used as methods that improve and outperform existing methods for diagnosis of hepatoma, by analysis weighted together with the AFP known as biomarkers for diagnosis of hepatoma.

## Claims

1. A composition for diagnosing or prognosing hepatoma, comprising;
a detection reagent of at least one biomarker selected from a group consisting of Insulin-like growth factor-binding protein complex acid labile subunit (ALS), Alpha-1-microglobulin/bikunin precursor (AMBP), Carboxypeptidase B2 (CPB2), Cholinesterase (CHLE), Clusterin (CLUS), Beta-Ala-His dipeptidase (CNDP1), Complement component C6 (CO6), Carboxypeptidase N subunit 2 (CPN2), Coagulation factor XI (FA11), Fibronectin (FINC), Hepatocyte growth factor activator (HGFA), Insulin-like growth factor-binding protein 3 (IBP3), Insulin-like growth factor II (IGF2), inter-alpha-trypsin inhibitor heavy chain H1 (ITIH1), Kallistatin (KAIN), Plasma kallikrein (KLKB1), Phosphatidylcholine-sterol acyltransferase (LCAT), Plasminogen-related protein A (PLGA), Plasminogen (PLMN), Prothrombin (THRB), Vitamin D binding protein (VTDB), and Vitronectin (VTNC).

2. The composition of Claim 1, wherein the biomarker is selected from a group consisting of ALS, CBPB2, CLUS, CNDP1, CPN2, FA11, FINC, and HGFA.

3. The composition of Claim 1, wherein the biomarker is a combination of FINC, THRB, and FINC; or a combination of ALS, ITIH1, THRB, and FINC.

4. The composition of Claim 1, wherein prognosing the hepatoma means determining whether recovery is achieved or not after the hepatoma has been treated

5. The composition of Claim 4, wherein the biomarker for determining whether the recovery is achieved or not after the hepatoma has been treated is selected from a group consisting of THRB, FINC, ITIH1, IBP3, PLMN, CBPB2, and PLGA.

6. The composition of Claim 1, of Claim 1, wherein the detection reagent detects the biomarker at a level of protein or a nucleic acid.

7. The composition of claim 6, wherein the detection reagent at the level of protein is a reagent for western blot, ELISA, radioimmunoassay (RIA), immunodiffusion, immunoelectrophoresis, immunohistochemistry (IHC), immunoprecipitation assays, complement fixation assays, FACS, mass spectrometry (MS), or protein microarray; and the detection reagent at the level of a nucleic acid is a reagent for nucleic acid amplification, polymerase chain reaction (PCR), reverse polymerase chain reaction, competitive polymerase chain reaction, nuclease protection assay (RNase, S1 nuclease assay), *in situ* hybridization, nucleic acid microarray or northern blot.

8. The composition of Claim 7, wherein the detection reagent at the level of protein includes an antibody, an antibody-binding fragment, an aptamer, an avidity multimer, a peptidomimetic, a receptor, a ligand or a cofactor that specifically recognizes full-length protein or a protein-binding fragment of the biomarker; and wherein the detection reagent at the level of a nucleic acid includes a primer or a probe that specifically recognizes a nucleic acid sequence of the biomarker, a nucleic acid sequence complementary to the former, and a fragment of the former or the latter

9. The composition for diagnosing or prognosing hepatoma of Claim 1, wherein the composition is used for ELISA, dipstick rapid kit analysis, MRM analysis, microarray, nucleic acid amplification (NAT), or immunoassay.

10. The composition of Claim 9, wherein the composition is used for the MRM analysis, and wherein peptides of each biomarker used for the MRM analysis are described in Tables 1-1 and 1-2.

11. The composition of any one of Claims 1 to 10, wherein the composition further includes an AFP biomarker in addition to the biomarker.

12. A method for detecting an *in vitro* biomarker of hepatoma to provide information required to diagnose or prognose the hepatoma, comprising steps of:
detecting presence and/or concentration of a nucleic acid and/or protein of at least one biomarker selected from a group consisting of Insulin-like growth factor-binding protein complex acid labile subunit (ALS), Alpha-1-microglobulin/bikunin precursor (AMBP), Carboxypeptidase B2 (CPB2), Cholinesterase (CHLE), Clusterin (CLUS), Beta-Ala-His dipeptidase (CNDP1), Complement component C6 (CO6), Carboxypeptidase N subunit 2 (CPN2), Coagulation factor XI (FA11), Fibronectin (FINC), Hepatocyte growth factor activator (HGFA), Insulin-like growth factor-binding protein 3 (IBP3), Insulin-like growth factor II (IGF2), inter-alpha-trypsin inhibitor heavy chain H1 (ITIH1), Kallistatin (KAIN), Plasma kallikrein (KLKB1), Phosphatidylcholine-sterol acyltransferase (LCAT), Plasminogen-related protein A (PLGA), Plasminogen (PLMN), Prothrombin (THRB), Vitamin D binding protein (VTDB), and Vitronectin (VTNC) from a biological sample derived from a subject;
comparing a result from detecting the level or the presence of the nucleic acid or the protein with a result corresponding to a biomarker of a sample of a control group; and
determining as the hepatoma if there is any change in the concentration or any change in a state of the presence of the nucleic acid or the protein of the sample of the subject in comparison with the sample of the control group.

13. The method of Claim 12, wherein the biological sample is whole blood, serum, or plasma; and the sample from the control group is a sample derived from a normal person or a patient that completely recovered from hepatitis, cirrhosis, or hepatoma.

14. The method of Claim 12, wherein the biomarker is selected from a group consisting of ALS, CBPB2, CLUS, CNDP1, CPN2, FA11, FINC, and HGFA.

15. The method of Claim 12, wherein the method further includes an AFP biomarker in addition to the at least one biomarker.

16. The method of Claim 12, wherein the method is conducted by using protein or nuclear acid microarray, nucleic acid amplification (NAT), antigen-antibody reaction, or mass spectrometry.

17. The method of Claim 12, wherein the diagnosis of the hepatoma includes the diagnosis of a patient with cirrhosis developing hepatoma and the prognosis of the hepatoma includes determining whether recovery is achieved or not after treatment of the hepatoma.
